(19) Europäisches Patentamt
European Patent Office
Office européen des brevets

(11) **EP 4 653 805 A1**

(12) **EUROPEAN PATENT APPLICATION**
published in accordance with Art. 153(4) EPC

(43) Date of publication:
**26.11.2025 Bulletin 2025/48**

(21) Application number: **24876082.9**

(22) Date of filing: **14.06.2024**

(51) International Patent Classification (IPC):
***G01B 7/16*** (2006.01)

(52) Cooperative Patent Classification (CPC):
**A41D 19/00; A61B 5/00; A61B 5/11; G01B 7/16;
G06F 3/01; G06V 40/20**

(86) International application number:
**PCT/CN2024/099232**

(87) International publication number:
**WO 2025/077257 (17.04.2025 Gazette 2025/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.10.2023 PCT/CN2023/124295**

(71) Applicant: **Shenzhen Shokz Co., Ltd.
Shenzhen, Guangdong 518108 (CN)**

(72) Inventors:
• **DENG, Wenjun
Shenzhen, Guangdong 518108 (CN)**
• **YUAN, Yongshuai
Shenzhen, Guangdong 518108 (CN)**

(74) Representative: **Wang, Bo
Panovision IP
Ebersberger Straße 3
85570 Markt Schwaben (DE)**

(54) **FORCE FEEDBACK SYSTEM**

(57) Embodiments of the present disclosure provides a force feedback system, including a glove body, a microprocessor coupled to the glove body and communicatively coupled to an external computing device, and a plurality of finger force feedback components. Each of the plurality of finger force feedback components is mechanically coupled to the glove body and communicatively coupled to the microprocessor, and is configured to provide, based on instructions from the microprocessor, force feedback to a finger corresponding to the finger force feedback component. The finger force feedback component includes a drawstring tracking a movement of the finger, a transmission member following a movement of the drawstring, and a stopping member. A plurality of stopping structures are sequentially disposed on the transmission member. The stopping member is configured to cooperate with any one of the plurality of stopping structures to brake the finger.

**FIG. 1**

**Description**

**CROSS REFERENCE TO RELATED APPLICATIONS**

The present disclosure claims priority to International Application No. PCT/CN2023/124295, filed on October 12, 2023, the entire contents of which are incorporated herein by reference.

**TECHNICAL FIELD**

The present disclosure relates to the technical field of electronic components, and in particular, to a force feedback system.

**BACKGROUND**

With the gradual maturity of AR/VR technologies and the emergence of the metaverse concept, in order to provide users with more realistic feedback, intelligent electronic devices for human-computer interaction are further required to possess mechanical perception and simulation capabilities. The fingers are the most critical part for determining mechanical perception and feedback in force feedback systems. However, to reproduce finger force feedback, the finger force feedback components are complex in structure and large in count, resulting in relatively high power consumption and large size of the finger force feedback components.

Therefore, there is necessary to develop a force feedback system that improves the efficiency of finger force feedback while reducing the power consumption and size of the finger force feedback components.

**SUMMARY**

One of the embodiments of this specification provides a force feedback system. The force feedback system includes a glove body; a microprocessor coupled to the glove body, wherein the microprocessor is communicatively coupled to an external computing device; and a plurality of finger force feedback components, wherein each of the plurality of finger force feedback components is mechanically coupled to the glove body and communicatively coupled to the microprocessor, and is configured to provide, based on instructions from the microprocessor, force feedback to a finger corresponding to the finger force feedback component. The finger force feedback component includes a drawstring configured to track a movement of the finger, a transmission member configured to follow a movement of the drawstring, and a stopping member. A plurality of stopping structures are sequentially disposed on the transmission member. The stopping member is configured to cooperate with any one of the plurality of stopping structures to brake the finger.

In some embodiments, the finger force feedback component includes a housing located on a backside of a hand, the transmission member and the stopping member are disposed within the housing, and the force feedback system further includes a fingertip sleeve corresponding to the finger force feedback component, the fingertip sleeve being mechanically connected to the transmission member through the drawstring.

In some embodiments, the finger force feedback component also includes a wire groove disposed between finger joints of the glove body, and the drawstring passes through the wire groove to connect the fingertip sleeve and the transmission member.

In some embodiments, the transmission member comprises a spool, the drawstring is wrapped around the spool, a surface of the spool is provided with the plurality of stopping structures, and the stopping member includes a stopping sheet that is capable of being inserted into a groove between the plurality of stopping structures.

In some embodiments, a gradient on one side of a stopping structure in the plurality of stopping structures that abuts the stopping sheet is greater than a gradient on the other side of the stopping structure.

In some embodiments, a tooth spacing of the plurality of stopping structures is less than 2 mm.

In some embodiments, the tooth spacing of the plurality of stopping structures is less than 1 mm.

In some embodiments, an inner surface of the housing that is opposite to the plurality of stopping structures is provided with a limiting protrusion.

In some embodiments, a height of the limiting protrusion is greater than a height of the plurality of stopping structures.

In some embodiments, the stopping member includes an transmission rod configured to press or release the stopping sheet in response to an instruction, wherein when the transmission rod presses the stopping sheet, the stopping sheet is inserted into the groove between the plurality of stopping structures.

In some embodiments, the stopping member comprises a piezoelectric structure configured to drive the stopping sheet to insert into the groove between the plurality of stopping structures.

In some embodiments, the stopping sheet corresponds to a plurality of depths when inserted into the groove between the plurality of stopping structures.

In some embodiments, the finger force feedback component further includes a sensing member configured to detect a movement distance of the drawstring and transmit data relating to the movement distance of the drawstring to the microprocessor.

In some embodiments, the transmission member comprises a spool, the drawstring is wrapped around the spool, and the sensing member comprises a magnet mechanically connected to the spool and a magnetic field sensor configured to measure rotation information of the magnet as the spool rotates.

In some embodiments, the magnet is arranged coaxially with the magnetic field sensor along a central axis of a rotation of the spool.

In some embodiments, the magnet is mechanically connected to a side surface of the spool, and the side surface is arranged with a limiting protrusion.

In some embodiments, a height of the limiting protrusion is greater than a height of the magnet.

In some embodiments, the finger force feedback component further includes strain sensors, and the strain sensors are arranged at finger joints of the glove body and configured to read posture data of the finger.

In some embodiments, the transmission member includes a spool and a spiral spring mechanically connected to the spool, the drawstring is wound around the spool, the spiral spring is configured to drive the spool to rotate in an opposite direction to reset the drawstring after the finger releases an external pulling force.

In some embodiments, a stiffness coefficient of the spiral spring is less than 100 N/m.

BRIEF DESCRIPTION OF THE DRAWINGS

The present disclosure will be further described by way of exemplary embodiments, which are described in detail with reference to the accompanying drawings. These embodiments are not limiting, and in these embodiments, the same numbering denotes the same structure, wherein:

FIG. 1 is a block diagram illustrating an exemplary force feedback system according to some embodiments of the present disclosure;

FIG. 2 is a schematic diagram illustrating an exemplary microprocessor according to some embodiments of the present disclosure;

FIG. 3 is a schematic diagram illustrating an exemplary communicative coupling between a microprocessor and an external computing device according to some embodiments of the present disclosure;

FIG. 4A is a schematic diagram illustrating an exemplary force feedback system according to some embodiments of the present disclosure;

FIG. 4B is a schematic diagram illustrating a side view of the force feedback system shown in FIG. 4A;

FIG. 5A is a schematic diagram illustrating a partial structure of an exemplary finger force feedback component according to some embodiments of the present disclosure;

FIG. 5B is a schematic diagram illustrating a side view of a partial structure of an exemplary stopping member according to some embodiments of the present disclosure;

FIG. 6A is a schematic diagram illustrating another exemplary partial structure of a finger force feedback component according to some embodiments of the present disclosure;

FIG. 6B is a schematic diagram illustrating a side view of another exemplary partial structure of a stopping member according to some embodiments of the present disclosure;

FIG. 7A is a schematic diagram illustrating another exemplary partial structure of a finger force feedback component according to some embodiments of the present disclosure;

FIG. 7B is a schematic diagram illustrating a side view of another exemplary partial structure of a stopping member according to some embodiments of the present disclosure;

FIG. 8 is a schematic diagram illustrating a side view of yet another exemplary partial structure of a stopping member according to some embodiments of the present disclosure;

FIG. 9 is a schematic diagram illustrating joint positions of a hand of a user according to some embodiments of the present disclosure;

FIG. 10 is a schematic diagram illustrating an exemplary hand motion coordinate system according to some embodiments of the present disclosure;

FIG. 11 is a schematic diagram illustrating a second strain sensor according to some embodiments of the present disclosure;

FIG. 12 is a schematic diagram illustrating another second strain sensor according to some embodiments of the present disclosure;

FIG. 13A is a schematic diagram illustrating a distribution of four capacitive structures on a flexible substrate according to some embodiments of the present disclosure;

FIG. 13B is a schematic diagram illustrating a cross-sectional view of the four capacitive structures shown in FIG. 13A along Y-axis;

FIG. 14A is a schematic diagram illustrating an undeformed state of a second sensor according to some embodiments of the present disclosure;

FIG. 14B is a schematic diagram illustrating deformation of the second sensor shown in FIG. 14A after being stretched or compressed along a long axis;

FIG. 15 is a schematic diagram illustrating a side view of the second sensor shown in FIG. 14A after being bent around an axis parallel to a short axis;

FIG. 16 is a schematic diagram illustrating a top view of the second sensor shown in FIG. 14A after being bent around an axis parallel to a thickness direction; and

FIG. 17 is a schematic diagram illustrating a cross-sectional view of a second strain sensor according to some embodiments of the present disclosure.

## DETAILED DESCRIPTION

In order to more clearly illustrate the technical solutions of the embodiments of the present disclosure, a brief introduction to the drawings required for the description of the embodiments is provided below. Obviously, the drawings described below are merely some examples or embodiments of the present disclosure, and a person of ordinary skill in the art may apply the present disclosure to other similar scenarios based on these drawings without creative efforts. Unless otherwise apparent from the context or explicitly stated, identical reference numerals in the drawings represent identical structures or operations.

It should be understood that the terms "system," "device," "unit," and/or "module" used herein are methods for distinguishing different levels of components, elements, parts, portions, or assemblies. However, the words may be replaced by other expressions in response to other words accomplish the same purpose.

As used in the disclosure and the appended claims, the singular forms "a," "an," and "the" include plural referents unless the content clearly dictates otherwise. Generally, the terms "include" and "comprise" merely indicate the inclusion of the explicitly identified steps or elements, and such steps or elements do not constitute an exclusive listing. The manner or device may also include other steps or elements.

Flowcharts are used in the present disclosure to illustrate operations performed by a system according to embodiments of the present disclosure. It should be understood that preceding or subsequent operations are not necessarily performed in the precise order presented. Instead, the steps may be performed in reverse order or concurrently. Additionally, other operations may be added to or one or more operations may be removed from these processes.

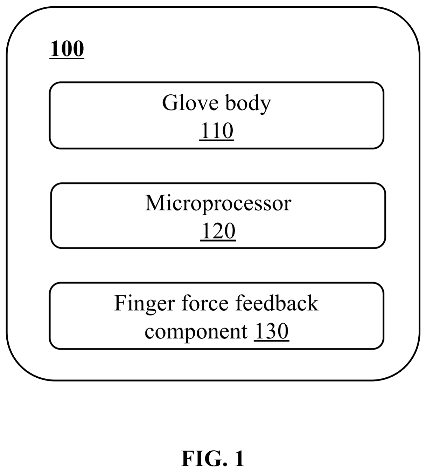

FIG. 1 is a block diagram illustrating an exemplary force feedback system according to some embodiments of the present disclosure. In some embodiments, as shown in FIG. 1, the force feedback system 100 may include a glove body 110, a microprocessor 120, and a finger force feedback component 130.

The force feedback system 100 refers to a system configured to provide a user with a reaction force from a target object in a virtual environment. The force feedback system 100 may perceive a behavior of the user's hand actions (e.g., grabbing, stroking, touching, slapping, or the like) on the target object, and simulate a corresponding reaction force to feed back to the user, assisting the user in perceiving the target object in the virtual environment through force and/or tactile sensations. The force feedback system 100 may feed back the reaction force corresponding to the behavior to the user through one or more devices, such as a movement capture glove, a VR glove, a force feedback glove, a haptic feedback glove, etc.

The glove body 110 refers to a wearable component configured to fit closely against a hand. In some embodiments, to facilitate close fitting between the glove body 110 and hand joints (e.g., including interphalangeal joints, metacarpophalangeal joints, wrist joints, or the like), the glove body 110 has a structure adapted to the shape of the hand and extending along the hand joints. In some embodiments, the glove body 110 may be made of a flexible fabric that is prone to deformation (such as bending deformation) in response to an external force and is able to follow movements of fingers. In some embodiments, the glove body 110 may provide support for the finger force feedback component 130 to facilitate the arrangement of the finger force feedback component 130. For example, the glove body 110 may include one or more layers of flexible structures (e.g., flexible fabric). The finger force feedback component 130 may be fixed to a surface of one layer of the flexible structure by means of adhesion, pressing, or stitching.

The finger force feedback component 130 refers to a component configured to provide feedback regarding a reaction force of a target object in a virtual environment to a finger of a user.

In some embodiments, the force feedback system 100 may include a plurality of finger force feedback components 130. For example, each finger may correspond to one finger force feedback component 130. In some embodiments, the finger force feedback component 130 may be mechanically coupled to the glove body 110. For example, each finger force feedback component 130 may be mechanically coupled to a position on a backside of a hand of the glove body 110 near a base of a corresponding finger. Merely by way of example, each finger force feedback component 130 may be fixed to the backside of a hand of the glove body 110 at a position near the base of the finger by means of adhesion, pressing, stitching, or the like.

In some embodiments, the finger force feedback component 130 may be communicatively coupled to the microprocessor 120 and may provide force feedback to a corresponding finger based on instructions from the microprocessor 120. In some embodiments, the finger force feedback component 130 may generate sensing signals and transmit the sensing signals to the microprocessor 120. The microprocessor 120 may further generate instructions for controlling the finger force feedback component 130 based on the sensing signals, thereby enabling interaction with the finger force feedback component 130.

In some embodiments, each finger force feedback component 130 comprises a drawstring tracking a movement of the finger, a transmission member following a movement of the drawstring, and a stopping member. In some embodiments, a plurality of stopping structures may be sequentially disposed on the transmission member. The stopping member may be configured to cooperate with any one of the plurality of stopping structures to brake the finger. For example, the transmission member may include a spool, the drawstring may be wound around the spool and drive the spool to rotate under an external pulling force. The plurality of stopping structures may include ratchets arranged on the spool, and the stopping member may include a stopping sheet that is capable of being inserted into a groove between the ratchets. When the stopping sheet is inserted into the groove between the ratchets, rotation of the spool may be obstructed, thereby achieving a braking effect. The drawstring is connected to a fingertip sleeve, so that a braking force applied to the spool by the stopping sheet may be transmitted to the fingertip sleeve, allowing the finger of the user to perceive a feedback force generated by the braking.

In some embodiments, the finger force feedback component 130 may include a sensing member. The sensing member may detect a movement state of the drawstring (e.g., a movement direction, a movement speed, a movement distance, or the like) and transmit data related to the movement state of the drawstring to the microprocessor 120. The microprocessor 120 may transmit data related to the movement state of the drawstring to an external computing device communicatively coupled to the microprocessor 120. The external computing device may determine a movement condition of the finger based on the data related to the movement state of the drawstring and generate corresponding information and/or control signals based on the determination result. The information and/or control signals may be further transmitted to the microprocessor 120 to control operations of the finger force feedback component 130.

In some embodiments, the finger force feedback component 130 may include a strain sensor. The strain sensor may be arranged at finger joint of the glove body 110 and may be configured to read posture data of the finger.

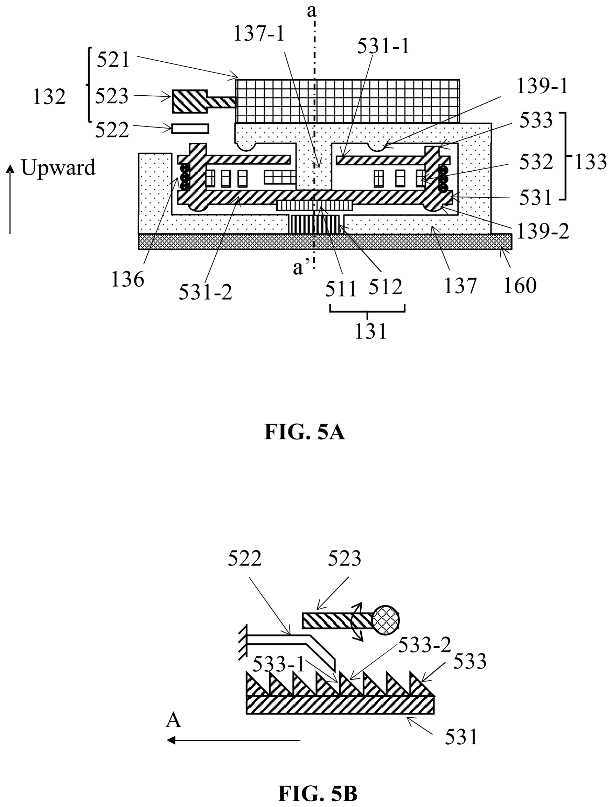

More descriptions regarding the detailed structure of the finger force feedback component 130 may be found in FIGs. 5A-8 and related descriptions thereof.

The microprocessor 120 may be communicatively coupled to the external computing device and to each finger force feedback component 130, and may process data and/or information obtained from each finger force feedback component 130 and/or the external computing device, thereby enabling interaction between the external computing device and the finger force feedback component 130. The external computing device may include a computer, a mobile phone, an AR/VR device, a robot, or the like. The interaction may include, for example, force feedback, controlling power on/off of the external computing device, volume adjustment, program processes of the external computing device (e.g., controlling movement of a game character), fitness feedback, or the like. In some embodiments, the microprocessor 120 may be coupled to the glove body 110. For example, the microprocessor 120 may be fixed to a surface of one layer of a flexible structure of the glove body 110 (e.g., fixed to a backside of a hand of the glove body 110) by means of adhesion, pressing, or stitching. In some embodiments, data related to the finger force feedback component 130 may be transmitted to the microprocessor 120 via a wire. In some embodiments, the microprocessor 120 may include one or more processing chips configured to analyze and process the received data. In some embodiments, the microprocessor 120 may be an independent processing device. In some embodiments, the microprocessor 120 may be an external device or a part of the glove body 110. For example, the microprocessor 120 may be integrated into the external computing device or into the glove body 110.

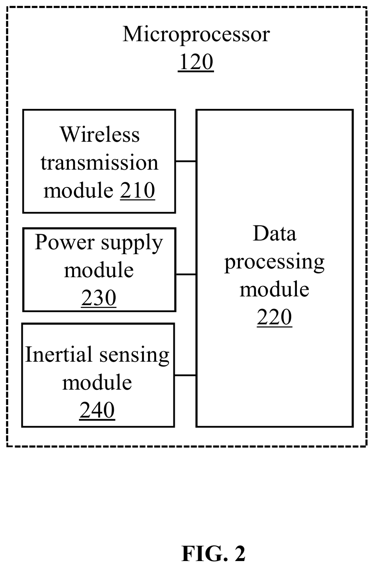

FIG. 2 is a schematic diagram illustrating an exemplary microprocessor according to some embodiments of the present disclosure.

As shown in FIG. 2, the microprocessor 120 may include a data processing module 220, a wireless transmission module 210, a power supply module 230, and an inertial sensing module 240.

The wireless transmission module 210 may be configured to communicate with an external computing device (e.g., a computer, an AR/VR host, a robot, or the like). For example, the wireless transmission module 210 may transmit data related to the finger force feedback component 130 (e.g., data related to a movement distance of the drawstring, posture data of the finger, or the like) to the external computing device via the network. As another example, the wireless transmission module 210 may also transmit data related to the inertial sensing module 240 (e.g., data related to spatial movement of the entire hand of the user) to the external computing device via the network. As another example, the wireless transmission module 210 may obtain a control signal from the external computing device via the network, the control signal being used to control the finger force feedback component 130. In some embodiments, the network may include a public network (e.g., the Internet), a private network (e.g., a local area network (LAN), a wide area network (WAN), or the like), a wired network (e.g., an Ethernet network), a wireless network (e.g., a Wi-Fi network), a cellular network (e.g., a Long Term Evolution (LTE) network), a virtual private network (VPN), a satellite network, a telephone network, a router, a hub, a switch, a server computer, and/or any combination thereof.

The data processing module 220 may be configured to process data and/or information obtained from the external computing device and/or the finger force feedback component 130. For example, the data processing module 220 may receive and process signals sent by a sensing member of the finger force feedback component 130 and transmit the signals to the external computing device via the wireless transmission module 210. As another example, the data processing module 220 may generate instructions based on signals received through the wireless transmission module 210 and send the instructions to the finger force feedback component 130, so as to control an operation of a stopping member in the finger force feedback component 130 and achieve braking of the finger.

The power supply module 230 may be configured to supply power to the microprocessor 120 and/or the finger force feedback component 130. For example, the power supply module 230 may supply power to components such as the stopping member and the sensing member in the finger force feedback component 130. In some embodiments, the power supply module 230 may include at least one battery or a battery pack.

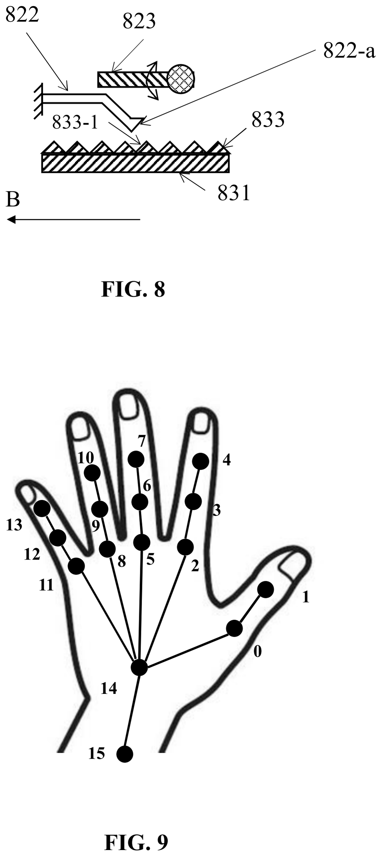

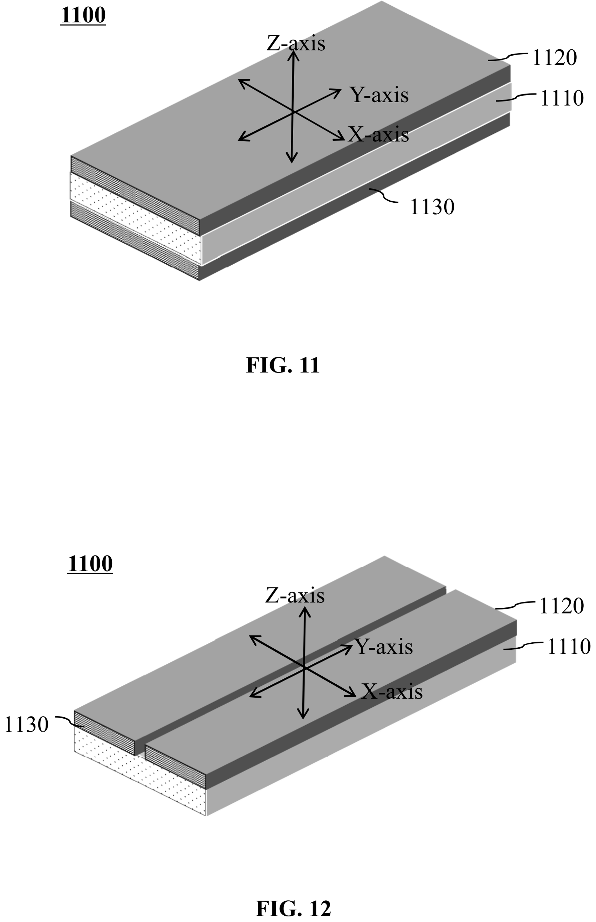

The inertial sensing module 240 may be configured to capture spatial movement of the entire hand of the user. In some embodiments, the spatial movement of the entire hand may be expressed as movement of the wrist joint of the hand, where the movement of the wrist joint may include three degrees of freedom: bending, swinging, and rotating. The bending and swinging of the wrist joint are controlled by the wrist joint and may cause deformation of the wrist joint. The rotation of the wrist joint is controlled by the rotation of the forearm of the user, resulting in overall rotation of the wrist joint. In some embodiments, in order to collect movement of the wrist joint in three degrees of freedom, the inertial sensing module 240 may include two inertial sensors. The two inertial sensors may be arranged on the glove body 110 and located on opposite sides of the wrist joint (e.g., positions 14 and 15 shown in FIG. 9). The opposite sides of the wrist joint refer to the front and rear sides of the wrist joint along an extension direction of the arm. The front side of the wrist joint refers to the side facing the back of the hand, and the rear side refers to the side facing the forearm. For ease of understanding, the opposite sides of the wrist joint may be regarded as regions of the hand or forearm that are close to the wrist joint in two directions but do not significantly deform with the deformation of the wrist joint. The two inertial sensors may respectively collect position information of the opposite sides of the wrist joint and cooperate to recognize movement of the wrist joint in three degrees of freedom, including overall rotation, bending, and swinging. In some embodiments, in order to collect movement of the wrist joint in three degrees of freedom, the inertial sensing module 240 may include an inertial sensor and a strain sensor. The inertial sensor and the strain sensor may cooperate to achieve movement acquisition of the wrist joint. In some embodiments, the strain sensor may include a multi-degree-of-freedom sensor and may be used to collect deformation of the wrist joint in multiple degrees of freedom. Merely by way of example, the inertial sensor and the strain sensor may be arranged on the glove body 110, where the strain sensor is located at the wrist joint and the inertial sensor is located outside the wrist joint region, such as on the front side or the rear side of the wrist joint. In some embodiments, the inertial sensor may be located on either side of the wrist joint along the extension direction of the arm. By using two inertial sensors or a combination of an inertial sensor and a strain sensor, movement of the wrist joint in multiple degrees of freedom may be collected. A user may flexibly select different combinations of sensor types according to different application scenarios to realize multi-degree-of-freedom movement acquisition. More descriptions regarding the multi-degree-of-freedom strain sensor may be found in other contents of the present disclosure (e.g., descriptions in connection with FIGs. 11-17).

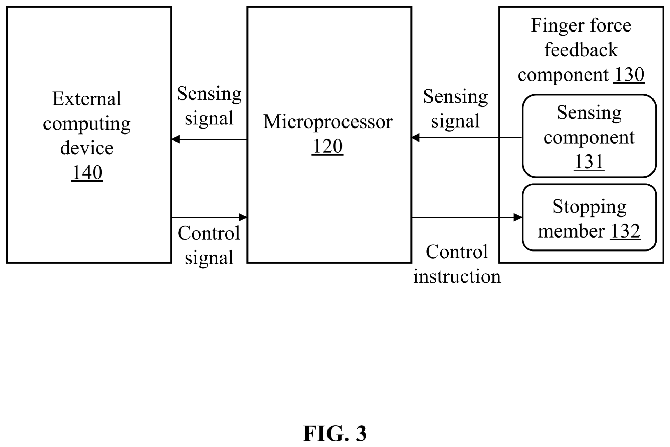

FIG. 3 is a schematic diagram illustrating an exemplary communicative coupling between a microprocessor and an external computing device according to some embodiments of the present disclosure.

As shown in FIG. 3, the microprocessor 120 may be communicatively coupled to the external computing device 140 and to each finger force feedback component 130, thereby enabling interaction between the external computing device 140 and the finger force feedback component 130. The interaction between the external computing device 140 and the finger force feedback component 130 may include sensing and force feedback.

During the sensing process between the external computing device 140 and the finger force feedback component 130, a sensing member 131 in the finger force feedback component 130 may generate a sensing signal based on a state of the finger force feedback component 130 and transmit the sensing signal to the microprocessor 120. For example, the state of the finger force feedback component 130 may include a movement state of the drawstring (e.g., a movement direction, a movement speed, a movement distance, or the like). The movement state of the drawstring may reflect a movement condition of a corresponding finger. For instance, when the finger bends, the drawstring is pulled and the movement distance of the drawstring gradually increases; when the finger is straightened or tilted upward, the drawstring resets and the movement distance of the drawstring gradually decreases. The sensing member 131 may detect the movement state of the drawstring (e.g., via a magnetic field) and generate data related to the movement state of the drawstring (i.e., the sensing signal) which and transmit the data to the microprocessor 120. As another example, the state of the finger force feedback component 130 may further include a posture of the finger, such as deformation of the finger in one or more degrees of freedom (bending, swinging, rotating, or the like). Merely by way of example, each finger force feedback component 130 may include a strain sensor, which is arranged at a finger joint of the glove body 110 and configured to read posture data of the finger and transmit the data to the microprocessor 120.

During the force feedback process between the external computing device 140 and the finger force feedback component 130, the external computing device 140 may determine a movement condition of the finger (e.g., whether deformation occurs, a type of deformation, a degree of deformation, or the like) based on the sensing signal and/or finger posture data, and may generate corresponding information and/or control signals based on the determination result. The information and/or control signals may be further transmitted to the microprocessor 120 to control an operation of the finger force feedback component 130. For example, the external computing device 140 may simulate a current posture of the hand based on the determination result of the finger movement condition and display the simulated posture on a display device. As another example, the external computing device 140 may determine whether braking should be applied to the finger based on the determination result of the finger movement condition and current scenario information, and generate a corresponding control signal The microprocessor 120 may generate a corresponding control instruction based on the control signal. The stopping member 132 and a transmission member (not shown) in the finger force feedback component 130 may cooperate to control braking or releasing of the drawstring based on the control instruction, so that the finger associated with the drawstring through a fingertip sleeve may perceive a force feedback.

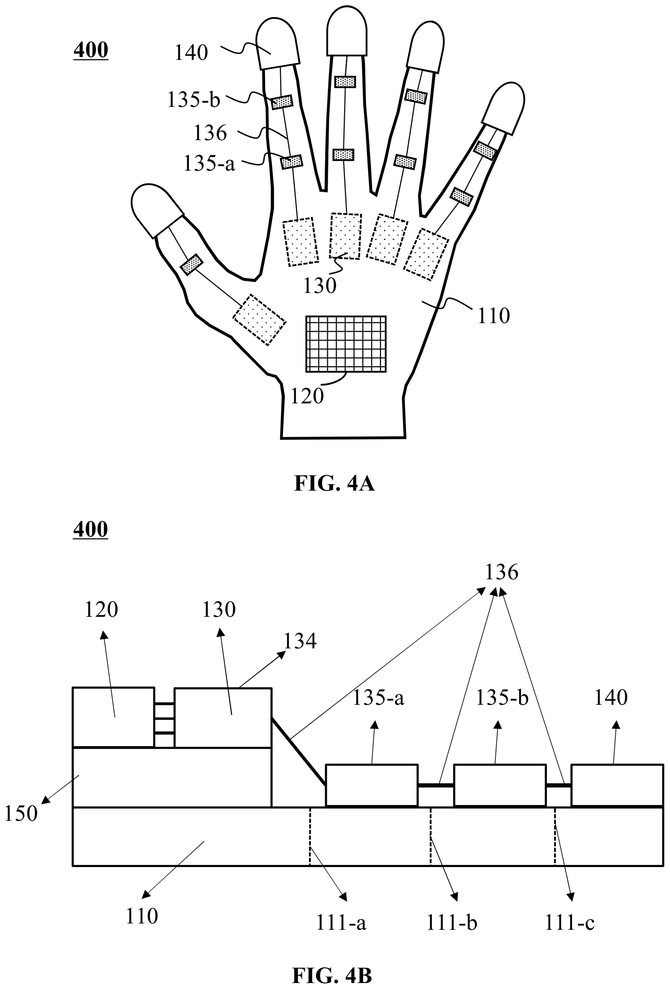

FIG. 4A is a schematic diagram illustrating an exemplary force feedback system according to some embodiments of the present disclosure; FIG. 4B is a schematic diagram illustrating a side view of the force feedback system shown in FIG. 4A.

In conjunction with FIGs. 4A and 4B, the force feedback system 400 may include a glove body 110, a microprocessor 120, a plurality of finger force feedback components 130, and fingertip sleeves 140. The microprocessor 120 and the finger force feedback components 130 may be coupled to the glove body 110. For example, the microprocessor 120 and the finger force feedback components 130 may be fixed to a backside of a hand of the glove body 110 via a base 150. The microprocessor 120 may be connected to the finger force feedback components 130 (e.g., to a circuit board in the finger force feedback component 130, not shown) via a wired or wireless connection, so as to transmit control instructions to the finger force feedback components 130 or read sensing signals and/or posture data generated by the finger force feedback components 130.

There may be a plurality of finger force feedback components 130 (e.g., five), each of which is configured to provide force feedback to a corresponding finger. Each finger force feedback component 130 may include a drawstring 136 tracking a movement of the finger, a transmission member (not shown) following a movement of the drawstring, and a stopping member (not shown). The finger force feedback component 130 may include a housing 134 located on a backside of a hand. The transmission member and the stopping member may be disposed within the housing 134. The drawstring 136 may be mechanically connected to the fingertip sleeve 140 and the transmission member inside the housing 134. As shown in FIG. 4A and FIG. 4B, the finger force feedback component 130 may include a wire groove disposed between finger joints of the glove body 110 (e.g., a wire groove 135-a disposed between a metacarpophalangeal joint 111-a and an interphalangeal joint 111-b, a wire groove 135-b disposed between the interphalangeal joint 111-b and another interphalangeal joint 111-c, or the like). The drawstring 136 may pass through the wire groove to connect the fingertip sleeve 140 and the transmission member.

In some embodiments, a plurality of stopping structures may be sequentially disposed on the transmission member. The stopping member may be configured to cooperate with any one of the plurality of stopping structures based on instructions from the microprocessor 120 to brake or release the drawstring 136. The drawstring 136 may further transmit a braking force of the finger force feedback component 130 to the fingertip sleeve 140, allowing a finger of the user to perceive a feedback force generated by the braking, thereby realizing force feedback of the force feedback system 100. The microprocessor 120 may also read sensing signals and/or posture data generated by the finger force feedback component 130, thereby determining a movement state or posture of the user finger.

FIG. 5A is a schematic diagram illustrating a partial structure of an exemplary finger force feedback component according to some embodiments of the present disclosure. In some embodiments, as shown in FIG. 5A, the finger force feedback component 130 may include a sensing member 131, a stopping member 132, a transmission member 133, a drawstring 136, and a rotating shaft frame 137. The transmission member 133 may be located inside the rotating shaft frame 137, and the stopping member 132 may be located outside the rotating shaft frame 137. Both the transmission member 133 and the stopping member 132 may be enclosed within a housing (e.g., the housing 134 shown in FIG. 4B). In some embodiments, the stopping member 132 may be disposed inside the rotating shaft frame 137, in which case the shaft frame 137 may serve as the housing.

The transmission member 133 may include a spool 531 and a spiral spring 532 mechanically connected to the spool 531. As shown in FIG. 5A, the rotating shaft frame 137 may be fixedly connected to a housing of the finger force feedback component 130 (e.g., the housing 134 shown in FIG. 4B), or the rotating shaft frame 137 itself may serve as the housing of the finger force feedback component 130. The spool 531 is disposed inside the rotating shaft frame 137. An upper side wall 531-1 of the spool 531 has an opening, and an inner wall of the rotating shaft frame 137 corresponding to the upper side wall 531-1 may include a spool post 137-1 extending toward the rotating shaft frame 137. The spool post 137-1 passes through the opening of the upper side wall 531-1 and extends into the spool 531, and is rotatably connected to a lower side wall 531-2 of the spool 531, such that the spool 531 is suspended inside the rotating shaft frame 137. The drawstring 136 is wound around the spool 531. When the drawstring 136 is pulled out by an external pulling force, the spool 531 may be driven by the drawstring 136 to rotate relative to the spool post 137-1, with a rotation axis as indicated by the dashed line aa' in the figure. When the finger force feedback component 130 is coupled to the glove body 110, an upper side wall 531-1 and a lower side wall 531-2 of the spool 531 are parallel or substantially parallel to a backside of a hand. The upper side wall 531-1 refers to a side wall of the spool 531 relatively far from the base, and the lower side wall 531-2 refers to a side wall of the spool 531 relatively close to the base. The rotation axis aa' may be perpendicular or substantially perpendicular to the backside of the hand. In some embodiments, to maintain stability of the spool 531 during rotation, the spool 531 may be configured as a cylindrical structure with the rotation axis aa' as a central axis. The opening on the upper side wall 531-1 may be located at a center of the upper side wall 531-1. A central axis of the spool post 137-1 may coincide with a central axis of the spool 531.

The spiral spring 532 may be mechanically connected to the spool 531 and configured to drive the spool 531 to rotate in an opposite direction to reset the drawstring 136 after the finger releases an external pulling force. As shown in FIG. 5A, the spiral spring 532 may be disposed inside the spool 531, with one end connected to the spool post 137-1 and the other end connected to an inner wall of the spool 531. When an external pulling force is applied by the finger, the drawstring 136 is pulled out and drives the spool 531 to rotate, and the spool 531 further tightens the spiral spring 532 through rotation. After the external pulling force is released by the finger, the spiral spring 532 may drive the spool 531 to rotate in an opposite direction to reset the drawstring 136.

In some embodiments, a stiffness coefficient of the spiral spring 532 may be set to adjust a restoring force of the spiral spring 532. If a stiffness coefficient of the spiral spring 532 is too small, the restoring force may be insufficient, which may hinder the resetting of the drawstring 136 and affect the subsequent braking effect on the drawstring. If the stiffness coefficient of the spiral spring 532 is too large, a significant increase in resistance during finger bending may occur, which may affect user experience. In addition, an excessively large stiffness coefficient may also cause the spool 531 to rotate too fast or rotate unstably after the external force applied by the finger is released, thereby affecting the sensing process of the sensing component. Therefore, in some embodiments, the stiffness coefficient of the spiral spring 532 may be less than 100 N/m, so as to effectively reset the drawstring 136 while improving user experience.

In some alternative embodiments, an elastic drawstring may be adopted to implement the aforementioned resetting. An exemplary elastic drawstring includes a rope made of rubber or elastic fibers. The restoring force of the elastic drawstring is similar to the restoring force provided by the spiral spring 532. Specifically, when an external pulling force is applied by a finger, the elastic drawstring is stretched, driving the spool 531 to rotate; when the external pulling force is released, the elastic drawstring returns to its original length using its own resilience. A stiffness coefficient of the elastic drawstring may be set to adjust the restoring force of the elastic drawstring, so as to avoid an increased sense of resistance during finger bending caused by an excessively large stiffness coefficient, or a failure to return to the original length due to an excessively small stiffness coefficient. In some embodiments, the stiffness coefficient of the elastic drawstring may be less than 60 N/m.

FIG. 5B is a schematic diagram illustrating a side view of a partial structure of an exemplary stopping member according to some embodiments of the present disclosure. In conjunction with FIG. 5A and FIG. 5B, a plurality of stopping structures 533 (e.g., ratchets, or the like) are sequentially arranged on a surface (or upper surface) of the spool 531. When the drawstring 136 drives the spool 531 to rotate, the stopping structures 533 rotate along with the spool 531. Merely by way of example, a rotation direction of the stopping structures 533 may be direction A. The stopping member 132 may be configured to cooperate with the stopping structures 533 to brake of the finger (or the drawstring 136). Specifically, the stopping member 132 may include an actuator 521, a stopping sheet 522, and a transmission rod 523.

The actuator 521 is configured to perform movement based on a control instruction and drive the transmission rod 523 to move. As illustrated in FIG. 5A, the actuator 521 may be disposed on the rotating shaft frame 137, for example, fixedly connected to an upper surface of the rotating shaft frame 137. One end of the transmission rod 523 is connected to the actuator 521, and the other end (also referred to as a free end) may be suspended. When the actuator 521 receives the control instruction from the microprocessor 120, the actuator 521 may move based on the control instruction and transmit the movement to the transmission rod 523. The transmission rod 523 may further convert the movement of the actuator 521 into an upward or downward movement, so that the free end of the transmission rod 523 is lifted or dropped. The free end of the transmission rod 523 may be suspended above the stopping sheet 522. When the free end of the transmission rod 523 is dropped, the movement of the actuator 521 may be transmitted to the stopping sheet 522, so that the stopping sheet 522 is inserted into a groove between the plurality of the stopping structures 533, thereby pressing the stopping sheet 522 according to the control instruction. In some embodiments, the actuator 521 may be a micro servo motor, or may be an electric component such as a piezoelectric sheet, a telescopic motor, an electromagnet, etc.

The stopping sheet 522 refers to a structure that is capable of being inserted into a groove between the stopping structures 533. In some embodiments, the stopping sheet 522 may be an elastic component. For example, the stopping sheet 522 may be an elastic spring sheet or a rigid component with a spring structure. One end of the stopping sheet 522 may be connected to the rotating shaft frame 137 (e.g., fixedly connected or rotatably connected), and the other end (also referred to as the free end) may extend in a curved manner toward the stopping structures 533 and be suspended above the stopping structures 533. A position of the stopping sheet 522 when not subjected to a force from the transmission rod 523 may be referred to as a resting position. When the stopping sheet 522 is at the resting position, the spool 531 may rotate in direction A under a force applied by the drawstring 136. When the transmission rod 523 (e.g., the free end) moves downward under an action of the actuator 521, the stopping sheet 522 moves downward under the pressure from the transmission rod 523, and the free end of the stopping sheet 522 is inserted into a groove between the stopping structures 533, thereby achieving an effect of restricting a movement of the stopping structures 533 (i.e., a braking effect). When the transmission rod 523 moves upward, the pressure applied to the stopping sheet 522 is released, and the stopping sheet 522 returns upward to the resting position under the action of its own elasticity, so that the stopping structures 533 are released and the spool 531 is allowed to rotate normally.

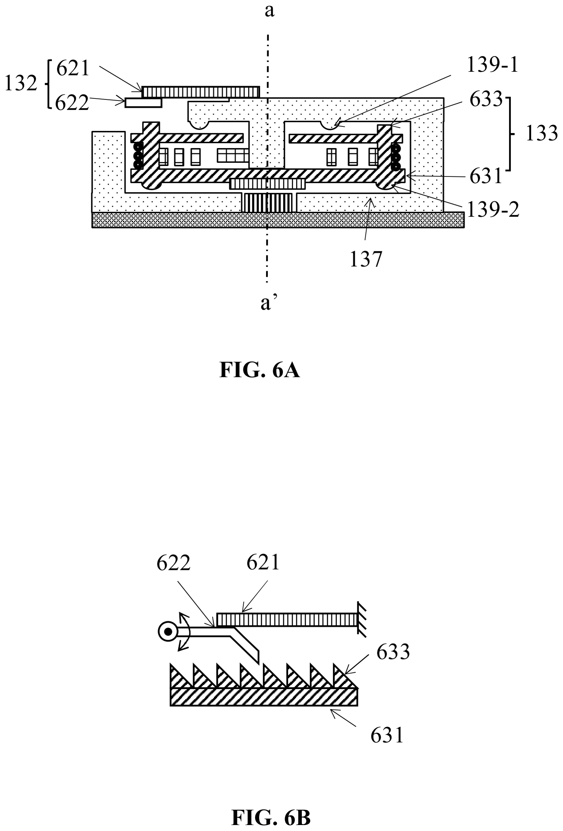

FIG. 6A is a schematic diagram illustrating another exemplary partial structure of a finger force feedback component according to some embodiments of the present disclosure. The finger force feedback component illustrated in FIG. 6A is substantially the same as the finger force feedback component illustrated in FIG. 5A, except that a braking effect may be achieved through a piezoelectric structure in the finger force feedback component illustrated in FIG. 6A. As illustrated in FIG. 6A, the stopping member 132 may include a piezoelectric structure 621.

The piezoelectric structure 621 may be a component that generates movement in response to a driving voltage. Specifically, the driving voltage acts on the piezoelectric layer of the piezoelectric structure 621, causing the piezoelectric layer to deform, thereby causing the piezoelectric structure 621 to move. In some embodiments, the piezoelectric layer may be formed of a piezoelectric material. Exemplary piezoelectric materials may include piezoelectric ceramics, piezoelectric crystals (e.g., barium titanate, lead zirconate titanate), piezoelectric polymers (e.g., polyvinylidene fluoride), etc., or any combination thereof. In some embodiments, the piezoelectric structure 621 may be of any shape, such as a sheet-shaped structure, a block-shaped structure, a columnar structure, a ring-shaped structure, or any combination thereof.

FIG. 6B is a schematic diagram illustrating a side view of a stopping member shown in FIG. 6A. In conjunction with FIGs. 6A and 6B, the piezoelectric structure 621 may be a cantilever beam with one fixed end, wherein one end (i.e., a fixed end) is fixedly supported on the rotating shaft frame 137, and the other end (i.e., a free end) is suspended above the stopping sheet 622 or connected to the stopping sheet 622. One end of the stopping sheet 622 may be connected to the rotating shaft frame 137 (e.g., through fixed connection or rotatable connection), and the other end (i.e., a free end) may extend in a curved manner toward the plurality of stopping structures 633 and suspended above the plurality of stopping structures 633. When the finger force feedback component 130 generates a driving voltage based on a control instruction to cause the piezoelectric structure 621 to undergo downward deformation, the stopping sheet 622 may be driven to move downward, such that the free end of the stopping sheet 622 is inserted into a groove between the plurality of stopping structures 633, thereby achieving an effect of restricting a movement of the spool 631. When the finger force feedback member 130 stops generating the driving voltage based on a control instruction, the piezoelectric structure 621 restores its shape and releases the stopping sheet 622. The stopping sheet 622 returns upward to a resting position. For example, one end of the stopping sheet 622 is fixedly connected to the rotating shaft frame 137, and a free end of the piezoelectric structure 621 is suspended above the stopping sheet 622. In this case, the stopping sheet 622 returns upward to the resting position under an effect of elastic force. As another example, one end of the stopping sheet 622 is rotatably connected to the rotating shaft frame 137, and the free end of the piezoelectric structure 621 is connected to the stopping sheet 622. In this case, the stopping sheet 622 is driven by the piezoelectric structure 621 to return upward to the resting position. Accordingly, the plurality of stopping structures 633 are released, and the spool 631 is allowed to rotate normally.

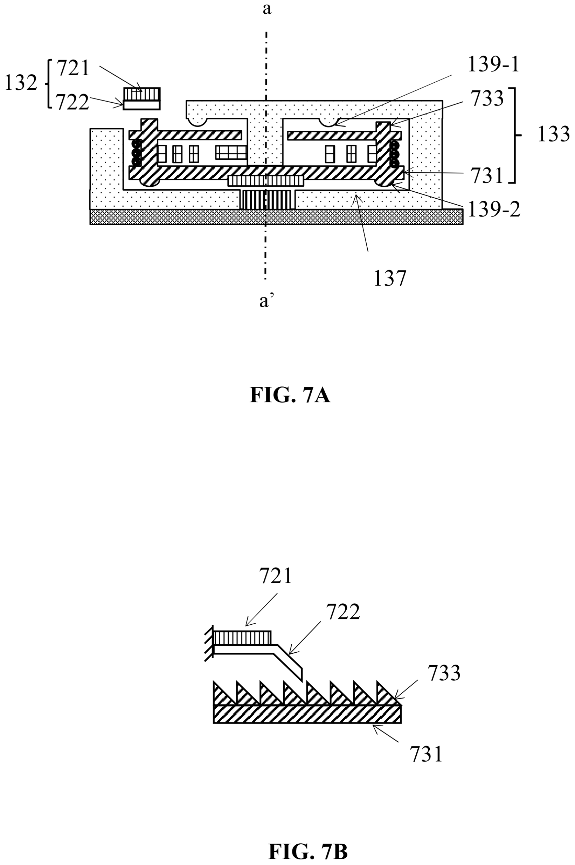

FIG. 7A is a schematic diagram illustrating another exemplary partial structure of a finger force feedback component according to some embodiments of the present disclosure. As illustrated in FIG. 7A, the stopping member 132 may include a piezoelectric structure 721. FIG. 7B is a schematic diagram illustrating a side view of another exemplary partial structure of a stopping member according to some embodiments of the present disclosure. In conjunction with FIGs. 7A and 7B, the piezoelectric structure 721 may be a piezoelectric sheet directly disposed on a surface of the stopping sheet 722. When the finger force feedback member 130 generates a driving voltage based on a control instruction to control the piezoelectric sheet to deform downward, the deformation of the piezoelectric sheet may drive a free end of the stopping sheet 722 to bend downward, such that the free end of the stopping sheet 722 is inserted into a groove between the plurality of stopping structures 733, thereby limiting a rotation of the spool 731. When the force feedback system 100 stops generating the driving voltage based on the control instruction, the piezoelectric sheet may restore its shape and drive the stopping sheet 722 to move upward, thereby releasing the stopping sheet 722 and allowing the spool 731 to rotate normally.

In some embodiments of the present disclosure, braking in cooperation with the stopping structures is achieved using the piezoelectric structure, such that a simple structure is achieved, an overall structure of the stopping member may be simplified, and production and assembly are facilitated. In addition, the piezoelectric structure may be a sheet structure with a relatively small size, which may reduce an overall size of the stopping member and the finger force feedback component, thereby facilitating a miniaturized design of the finger force feedback component.

In some embodiments, the gradient on one side of the stopping structure that abuts the stopping sheet may be greater than a gradient on the other side of the stopping structure. In some embodiments of the present disclosure, a gradient on one side of the stopping structure refers to a slope of the side. When the side is a plane, the slope may be represented by an included angle between the side and an upper side surface of the spool 531. When the side is a curved surface, the slope may be represented by an included angle between a tangent line of the side (e.g., a tangent at the contact point on the side where the stopping sheet abuts the side surface) and the upper side surface of the spool 531. For example, in connection with FIG. 5B, the stopping structures may include ratchets, and a gradient of one side 533-1 of a ratchet tooth of the ratchets, which abuts the stopping sheet 522, may be a right angle of 90°, and a gradient of the other side 533-2 may be an acute angle less than 90°. In some embodiments, a shape of a free end of the stopping sheet 522 may be matched with a gradient of the stopping structure 522. For example, the gradient of the free end of the stopping sheet 522 may also be a right angle of 90°. When the stopping sheet 522 is inserted into a groove between the ratchets, the free end of the stopping sheet 522 abuts the side 533-1 of the stopping structure 522. Since the gradient of the side 533-1 of the stopping structure 522 is relatively large, when the stopping sheet 522 abuts the side 533-1 of the stopping structure 522, it prevents the spool 531 from continuing to move in the direction A, thereby achieving rapid braking.

As described above, the use of ratchet teeth may enable a rapid switch between a braking state and a releasing state. However, the ratchet teeth may not support a transition between the braking state and the releasing state, and may not simulate braking forces of different magnitudes. Accordingly, different magnitudes of feedback force may not be provided. In some embodiments, the stopping sheet and the stopping structure may be configured such that the stopping sheet corresponds to a plurality of depths when inserted into a groove between teeth of the stopping structure, thereby providing different magnitudes of feedback force.

FIG. 8 is a schematic diagram illustrating a side view of yet another exemplary partial structure of a stopping member according to some embodiments of the present disclosure. As illustrated in FIG. 8, the stopping structure 833 may include a ratchet wheel, and the shape of an end 822-a of the stopping sheet 822 may match a shape of the ratchet wheel. By controlling a pressing force applied to the stopping sheet 822, different magnitudes of feedback force may be simulated. For example, in order to provide different magnitudes of feedback force, the stopping sheet 822 may be configured as an elastic structure. When the stopping sheet 822 abuts a side 833-1 of the stopping structure 833 to perform braking, a continued downward pressing force applied by the transmission rod 823 to the stopping sheet 822 may cause the stopping sheet 822 to deform due to its elasticity, and a larger deformation amount may correspond to a greater feedback force. As another example, the stopping sheet 822 may correspond to a plurality of depths when inserted into a groove between teeth of the ratchet wheel. The plurality of depths may correspond to a plurality of different magnitudes of feedback force, thereby enabling simulation of different feedback forces. Merely by way of example, when the stopping sheet 822 is in a static position, the stopping structure 833 may move along a direction B with the spool 831. When the stopping sheet 822 moves downward and is inserted into a groove between teeth of the ratchet wheel, an end 822-a of the stopping sheet 822 abuts a side 833-1 of the stopping structure 833, and resistance generated by the abutment provides a feedback force to a finger of the user. When a depth of insertion of the stopping sheet 822 into a groove between teeth of the ratchet wheel varies, an abutment angle between the stopping sheet 822 and the spool 831 differs accordingly, resulting in different magnitudes of feedback force provided to a finger of the user. Accordingly, different magnitudes of force feedback may be provided by controlling a depth at which the stopping sheet 822 is inserted into a groove between teeth of the ratchet wheel.

In some embodiments of the present disclosure, a transition state between braking and releasing can be established to better simulate the force feedback experienced when a finger grips or touches an object. For example, when a finger grips an elastic object, the force feedback gradually increases from initial contact to final grasp, and the transition state between braking and releasing can represent this process. In some embodiments, to enable the force feedback to better match expectations in real scenarios, a plurality of stopping members described above may be provided. The plurality of stopping members may be made of different materials or have different structures to exhibit different braking effects. For example, two stopping members with different stiffness may be formed from materials with different hardness. The stopping member with greater stiffness may achieve braking more quickly when cooperating with the stopping structure, whereas a stopping member with lower stiffness may achieve braking in a relatively slower manner when cooperating with the stopping structure, and compared with the stopping member with greater stiffness, may better represent a transition state in which the feedback force gradually increases. Similarly, stopping members with different structures (e.g., the stopping members shown in FIGs. 7B and 8) may also exhibit different braking effects. In practical applications, the stopping member used for braking may be flexibly selected according to the scenario to be simulated.

The tooth spacing may refer to a distance between two adjacent stopping structures. For example, the stopping structures may include a ratchet, and the tooth spacing may be a distance between tips of two adjacent teeth of the ratchet. In some embodiments, the tooth spacing may affect a control accuracy of the stopping member 132. For example, when the tooth spacing is small, the stopping sheet may quickly abut against the stopping structure during a downward movement, thereby enabling instant braking and improving the control accuracy of the stopping member 132. In some embodiments, the tooth spacing of the stopping structure may be less than 2 mm, thereby ensuring the control accuracy of the stopping member 132. In some embodiments, to further improve the control accuracy of the stopping member 132, the tooth spacing of the stopping structure may be less than 1 mm.

In some embodiments, as illustrated in FIG. 5A, an inner surface of the rotating shaft frame 137 that is opposite to the stopping structure 533 may be provided with a limiting protrusion 139-1. The limiting protrusion 139-1 may be a protruding structure configured to limit a position of a component. For example, the limiting protrusion 139-1 may be used to limit a distance between the inner surface of the rotating shaft frame 137 and the stopping structure 533, thereby preventing friction between the stopping structure 533 and the rotating shaft frame 137 from affecting an accuracy of the force feedback. In some embodiments, the limiting protrusion 139-1 may be arranged in a staggered manner with the stopping structure 533. For example, on a projection plane along a central axis aa' of the spool 531, the limiting protrusion 139-1 and the stopping structure 533 do not overlap. In some embodiments, the limiting protrusion 139-1 may be distributed around an inner surface of the rotating shaft frame 137. For example, a plurality of limiting protrusions 139-1 may be disposed on the inner surface of the rotating shaft frame 137 and the plurality of limiting protrusions 139-1 may be distributed around the central axis aa' of the spool 531. In some embodiments, the plurality of limiting protrusions 139-1 may be evenly distributed along a circle centered at a position of the central axis aa', on the inner surface of the rotating shaft frame 137, such that a distance between the inner surface of the rotating shaft frame 137 and the stopping structure 533 may be evenly limited, thereby preventing tilting of the spool 531 during rotation that may cause friction between the stopping structure 533 and the rotating shaft frame 137. It should be understood that the arrangement of the limiting protrusion 139-1 illustrated in FIG. 5A is merely exemplary. In some embodiments, the limiting protrusion 139-1 may be disposed at any position that can limit a distance between the inner surface of the rotating shaft frame 137 and the stopping structure 533. For example, the limiting protrusion 139-1 may be disposed on an upper surface of the spool 531, i.e., a plane where the stopping structure 533 is located.

In some embodiments, a height of the limiting protrusion 139-1 is greater than a height of the plurality of stopping structures 533, wherein the height of the limiting protrusion 139-1 refers to a length dimension of the limiting protrusion 139-1 in a direction along a central axis aa' of the spool 531. By setting the height of the limiting protrusion 139-1 to be greater than the height of the stopping structure 533, a relative position between the spool 531 and the rotating shaft frame 137 may be limited. For example, when the spool 531 rotates and deflects upward to contact the rotating shaft frame 137, since the limiting protrusion 139-1 has a height greater than that of the stopping structure 533, the limiting protrusion 139-1 may be in contact with an upper surface of the spool 531 where the stopping structure 533 is disposed before the stopping structure 533 contacts the rotating shaft frame 137, thereby preventing friction between the stopping structure 533 and the rotating shaft frame 137 and ensuring accuracy of force feedback. In some embodiments, a height of the limiting protrusion 139-1 may be less than a distance between an upper surface of the spool 531 and an inner surface of the rotating shaft frame 137, so as to reduce friction between the limiting protrusion 139-1 and the upper surface of the spool 531. In some embodiments, in order to prevent the friction between the limiting protrusion 139-1 and the upper surface of the spool 531 from affecting accuracy of force feedback, the limiting protrusion 139-1 may have a smooth surface structure.

In the embodiments of the present disclosure, the limiting protrusion 139-1 is exemplarily described as being disposed on an inner surface of the rotating shaft frame. In some embodiments, the limiting protrusion 139-1 may be disposed at other positions. For example, the limiting protrusion 139-1 may be disposed on an upper surface of the spool 531. As another example, a housing of the finger force feedback component 130 (e.g., the housing 134 shown in FIG. 2) may be used interchangeably with the rotating shaft frame. Merely by way of example, the stopping member 132 may be located inside the rotating shaft frame 137, in which case the rotating shaft frame 137 may serve as the housing, and the limiting protrusion 139-1 may be disposed on an inner surface of the housing opposite to the stopping structure.

As illustrated in FIG. 5A, each finger force feedback component further includes a sensing member 131. The sensing member 131 may be configured to detect a movement distance of the drawstring 136 and transmit data relating to the movement distance of the drawstring 136 to the microprocessor 120. The microprocessor 120 or the external computing device 140 may determine whether to brake the finger based on the data relating to the movement distance of the drawstring 136 and current scenario information, and generate a corresponding control signal.

The sensing member 131 may include a magnet 511 and a magnetic field sensor 512. The magnet 511 is mechanically connected to the spool 531 to rotate along with the spool. For example, the magnet 511 may be mechanically connected to an outer surface of a lower sidewall 531-2 of the spool 531 (i.e., a lower surface of the spool 531). The magnetic field sensor 512 may be disposed opposite to the magnet 511. For example, the magnetic field sensor 512 may be disposed on an inner surface of the rotating shaft frame opposite to the magnet 511. Alternatively, the magnetic field sensor 512 may be embedded in the inner surface of the rotating shaft frame opposite to the magnet 511 such that an upper surface of the magnetic field sensor 512 is coplanar with the inner surface of the rotating shaft frame. The magnetic field sensor 512 may be configured to measure rotation information of the magnet 511 as the spool 531 rotates. Merely by way of example, the magnet 511 and the magnetic field sensor 512 may both be cylindrical and coaxially arranged. When the drawstring 136 is pulled outward by an external pulling force or reset by rotation, the spool 531 may rotate around a central axis aa', and the magnet 511 may rotate together with the spool 531, causing a change in the magnetic field direction. The magnetic field sensor 512 may detect the magnetic field change and generate a sensing signal. The change may reflect rotation information of the spool 531, thereby indicating a movement state of the drawstring 136. For example, a change direction of the magnetic field may reflect a rotation direction of the spool 531, i.e., a movement direction of the drawstring 136 (pulling out or resetting). As another example, a rate of change in the magnetic field may reflect a rotation speed of the spool 531, i.e., a movement speed of the drawstring 136. As another example, a total change amount of the magnetic field may reflect a rotation degree of the spool 531, i.e., a movement distance of the drawstring 136. The movement state of the drawstring 136 may further reflect a movement state of the finger of the user. For example, whether the finger of the user is bent and a degree of bending may be determined based on the movement state of the drawstring 136.

In some embodiments, the finger force feedback component 130 may further include a printed circuit board (PCB) 160. The PCB 160 may be a component configured to read data generated by the finger force feedback component 130 (e.g., sensing signals generated by the sensing member 131, finger posture data generated by strain sensors, or the like) and/or receive control instructions input into the finger force feedback component 130. In conjunction with FIG. 5A, the PCB 160 may be disposed at a bottom of the finger force feedback component 130. The PCB 160 may be configured to read the data generated by the finger force feedback component 130 and transmit the data to the microprocessor 120, or receive control instructions from the microprocessor 120 to control the stopping member 132 to implement braking.

In some embodiments, in conjunction with FIG. 5A, the magnet 511 may be arranged coaxially with the magnetic field sensor 512 along a central axis of a rotation of the spool 531. Such an arrangement enables the magnet 511 to rotate synchronously with the spool 531 around the central axis, and the magnetic field sensor 512 may accurately sense magnetic field changes, thereby accurately and promptly detecting a movement state of the drawstring 136.

In conjunction with FIG. 5A, the magnet 511 is connected to a lower surface of the spool 531, and a limiting protrusion 139-2 may be disposed on the lower surface of the spool 531. The limiting protrusion 139-2 may be used to limit a distance between the lower surface of the spool 531 and an inner surface of the rotating shaft frame 137, thereby reducing or preventing friction between the lower surface of the spool 531 and the rotating shaft frame. The limiting protrusion 139-2 may further be used to limit a position of the magnet 511 relative to the magnetic field sensor 512, such that a distance is maintained between the magnet 511 and the magnetic field sensor 512, thereby preventing contact between the magnet 511 and the magnetic field sensor 512 that may affect magnetic field variation and sensing. In some embodiments, the limiting protrusion 139-2 may be staggered with the magnetic field sensor 512. For example, on a projection plane along a central axis aa' of the spool 531, the limiting protrusion 139-2 does not overlap with the magnetic field sensor 512. In some embodiments, the limiting protrusion 139-2 may be distributed around a lower surface of the spool 531. For example, a plurality of limiting protrusions 139-2 may be disposed on the lower surface of the spool 531, and the plurality of limiting protrusions 139-2 may be distributed around the central axis aa' of the spool 531. In some embodiments, the plurality of limiting protrusions 139-2 may be evenly distributed along a circular shape on a lower surface of the spool 531, the circular shape being centered on a position of the central axis aa', such that a position of the lower surface of the spool 531 relative to the magnetic field sensor 512 may be evenly constrained, thereby preventing tilting of the spool 531 during rotation that may cause contact between the magnet 511 and the magnetic field sensor 512. It should be noted that the arrangement position of the limiting protrusions 139-2 shown in FIG. 5A is merely for illustration. In some embodiments, the limiting protrusions 139-2 may also be disposed on an inner surface of the rotating shaft frame 137 opposite to the magnet 511.

In some embodiments, a height of the limiting protrusion 139-2 may be greater than a height of the magnet 511. The height of the limiting protrusion 139-2 and the height of the magnet 511 refer to length dimensions of the limiting protrusion 139-2 and the magnet 511 along a direction of the central axis aa' of the spool 531. In some embodiments of the present disclosure, by setting the height of the limiting protrusion 139-2 to be greater than the height of the magnet 511, a position of a lower surface of the spool 531 relative to the magnetic field sensor 512 may be constrained, such that a distance is maintained between the magnet 511 and the magnetic field sensor 512, thereby preventing contact between the magnet 511 and the magnetic field sensor 512 that may affect magnetic field variation and sensing. For example, as illustrated in FIG. 5A, the magnetic field sensor 512 may be embedded in an inner surface of the rotating shaft frame that is opposite to the magnet 511, such that an upper surface of the magnetic field sensor 512 is coplanar with the inner surface of the rotating shaft frame 137 opposite to the magnet 511. When the spool 531 rotates and deflects downward, resulting in the magnet 511 to be in contact with the magnetic field sensor 512 (or the inner surface of the rotating shaft frame 137), the limiting protrusion 139-2, having a height greater than a height of the magnet 511, may first be in contact with the inner surface of the rotating shaft frame 137, so as to maintain a distance between the magnet 511 and the magnetic field sensor 512, thereby preventing contact between the magnet 511 and the magnetic field sensor 512 from affecting magnetic field variation and sensing. In some embodiments, a height of the limiting protrusion 139-2 may be less than a distance between a lower surface of the spool 531 and the inner surface of the rotating shaft frame 137, thereby reducing friction between the limiting protrusion 139-2 and the inner surface of the rotating shaft frame 137. In some embodiments, to prevent the friction between the limiting protrusion 139-2 and the inner surface of the rotating shaft frame 137 from affecting the accuracy of force feedback, the limiting protrusion 139-2 may be configured as a smooth-surfaced structure.

In the embodiments of the present disclosure, the limiting protrusion 139-2 is exemplarily described as being disposed on a lower surface of the spool 531. In some embodiments, the limiting protrusion 139-2 may be disposed in other positions. For example, the limiting protrusion 139-2 may be disposed on an inner surface of the rotating shaft frame (i.e., an inner surface opposite to the magnet 511). As another example, the housing of the finger force feedback component (e.g., the housing 134 shown in FIG. 2) may be interchangeably used with the rotating shaft frame. Merely by way of example, the stopping member 132 may be located inside the rotating shaft frame 137. In this case, the rotating shaft frame 137 may serve as the housing, and the limiting protrusion 139-2 may be disposed on an inner surface of the housing opposite to the magnet 511.

In some embodiments, the finger force feedback component 130 may include a strain sensor. In some embodiments, the strain sensor may be configured to read posture data of the finger. When the user wears the glove body 110 and moves a finger, a finger region of the glove body 110 undergoes deformation under an external force. The strain sensor is capable of converting deformation information at a finger joint (e.g., deformation direction, deformation magnitude, or the like) into an electrical signal. The electrical signal generated by the strain sensor is capable of reflecting posture data of a corresponding region of the finger. The microprocessor 120 or the external computing device 140 may be configured to determine whether to brake the finger and generate a corresponding control signal based on the electrical signal generated by the strain sensor and current scenario information. In some embodiments, the strain sensor may include, but not limited to, a capacitive strain sensor, an inductive sensor, a resistive sensor, an optical fiber sensor, or the like.

In some embodiments, the strain sensor may be arranged at finger joints of the glove body. For example, in conjunction with FIGs. 4A and 4B, the strain sensors may be disposed at a metacarpophalangeal joint 111-a, a proximal interphalangeal joint 111-b, and a distal interphalangeal joint 111-c. Further in conjunction with FIG. 9, FIG. 9 is a schematic diagram illustrating joint positions of a hand of a user according to some embodiments of the present disclosure. The finger joints of the hand may include metacarpophalangeal joints at positions 0, 2, 5, 8, and 11, and interphalangeal joints at positions 1, 3, 6, 9, 12, 4, 7, 10, and 13. In some embodiments, the finger force feedback component 130 may include a plurality of strain sensors disposed at the finger joints of the glove body 110. For example, the plurality of strain sensors may include first strain sensors disposed at the interphalangeal joints at positions 1, 3, 6, 9, 12, 4, 7, 10, and 13, the first strain sensors being configured to measure deformation of the interphalangeal joints in a single degree of freedom. As another example, the plurality of strain sensors may further include second strain sensors disposed at the metacarpophalangeal joints at positions 0, 2, 5, 8, and 11, the second strain sensors being configured to measure deformation of the metacarpophalangeal joints in two degrees of freedom. In some embodiments, the first strain sensors include single-degree-of-freedom sensors, such as uniaxial sensors (e.g., inductive sensors, resistive bending sensors, or capacitive tensile sensors). The second strain sensors include multi-degree-of-freedom sensors, such as multiaxial sensors (e.g., capacitive multiaxial motion sensors).

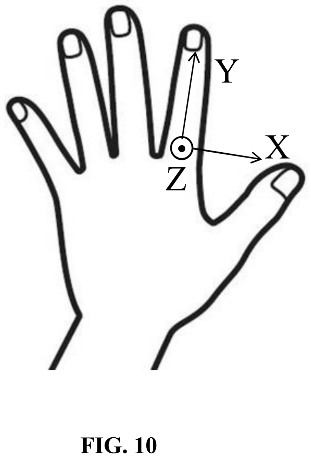

The degree of freedom described in the present disclosure refers to a dimension in which an object is capable of moving. To facilitate the description of the degrees of freedom of hand movement, a three-dimensional coordinate system is established for the movement of the hand, for example, the three-dimensional coordinate system illustrated in FIG. 10. In the coordinate system, a metacarpophalangeal joint is taken as the origin, a direction in which a finger extends is taken as a Y-axis, and a direction perpendicular to a hand plane is taken as a Z-axis. The hand plane may refer to a fitting surface of a palm portion. For example, when a palm of the hand is placed in close contact with a desktop, the desktop or a plane passing through the hand and parallel to the desktop may serve as the hand plane. The three-dimensional coordinate system may be used to describe the degrees of freedom of hand movement. Specifically, in the three-dimensional coordinate system illustrated in FIG. 10, rotation of the hand around the X-axis is defined as bending, rotation of the hand around the Z-axis is defined as swinging, and rotation of the hand around the Y-axis is defined as rotating. An interphalangeal joint can only perform bending and rotating. In other words, deformation of the interphalangeal joint only corresponds to the bending degree of freedom. The metacarpophalangeal joint can perform bending, swinging, and rotating. In other words, deformation of the metacarpophalangeal joint corresponds to the degrees of freedom including bending and swinging. Accordingly, when any interphalangeal joint performs a bending movement, a first strain sensor may measure a deformation occurring at a portion of the glove body 110 corresponding to the interphalangeal joint. When any metacarpophalangeal joint performs a bending or swinging movement, a second strain sensor may measure a deformation occurring at a portion of the glove body 110 corresponding to the metacarpophalangeal joint.

Referring further to FIG. 9, interphalangeal joints 4, 7, 10, and 13 are distal interphalangeal joints relatively farther from the palm, and interphalangeal joints 1, 3, 6, 9, and 12 are proximal interphalangeal joints relatively closer to the palm. In some embodiments, the movement of the distal interphalangeal joint is manifested as following the movement of the previous joint (i.e., the proximal interphalangeal joint), and does not bend independently but bends together with the proximal interphalangeal joint. Therefore, first strain sensors may be disposed only at the proximal interphalangeal joints without being disposed at the distal interphalangeal joints. In some embodiments, deformation data of the distal interphalangeal joints may be generated by mapping deformation data of the corresponding proximal interphalangeal joints. Accordingly, the force feedback system 100 may acquire deformation data in a single degree of freedom at five proximal interphalangeal joints through a plurality of first strain sensors, and acquire deformation data in two degrees of freedom at five metacarpophalangeal joints through a plurality of second strain sensors. In total, the plurality of first strain sensors and the plurality of second strain sensors collectively provide at least 15 degrees of freedom of deformation data of the user's fingers, thereby determining posture data of the fingers.

In some embodiments, the finger posture data acquired by the plurality of strain sensors may further be combined with movement data of a wrist joint of the user's hand acquired by an inertial sensing module 240 to jointly determine the posture of the user's hand.

In some embodiments, the first strain sensor is a single-axis sensor configured to acquire deformation of the interphalangeal joint in a single degree of freedom (i.e., bending).

In some embodiments, the first strain sensor includes various types of flexible bending sensors, such as inductive sensors, resistive sensors, capacitive sensors, optical fiber sensors, or the like. Merely by way of example, the first strain sensor includes an inductive sensor, and an inductance of the inductive sensor varies with deformation of the interphalangeal joint. Specifically, the inductive sensor deforms with the movement of the interphalangeal joint, and the inductance changes in response to the deformation of the inductive sensor. By acquiring parameters related to the inductance of the inductive sensor and analyzing their variation, the bending condition (e.g., bending angle, bending direction, etc.) of the inductive sensor may be accurately determined. For example, an electrical quantity of the inductance of the inductive sensor is proportional to a deformation amount of the inductive sensor. Accordingly, the change in the electrical quantity of the inductance of the inductive sensor may represent a deformation or bending degree of the interphalangeal joint. Since the interphalangeal joint corresponds to only one bending degree of freedom, using a single-axis sensor (e.g., an inductive sensor) to measure movement of the interphalangeal joint provides a simple structure and low product cost. Additionally, the first strain sensor may be arranged only at the interphalangeal joint of each finger that is closest to the corresponding metacarpophalangeal joint, thereby simplifying a structure of the finger force feedback component, reducing product cost, and ensuring the accuracy of deformation data acquisition.

In some embodiments, the second strain sensor is a multi-axis sensor configured to acquire deformation of the metacarpophalangeal joint in two degrees of freedom (i.e., bending and swinging). In some embodiments, the second strain sensor includes various types of flexible bending sensors, such as inductive sensors, resistive sensors, capacitive sensors, optical fiber sensors, or the like. Merely by way of example, the second strain sensor includes a plurality of capacitive structures, and capacitance values of the plurality of capacitive structures vary respectively with changes of the metacarpophalangeal joint in the respective degrees of freedom.

Referring to FIG. 11 and FIG. 12, the second strain sensor 1100 includes a flexible substrate 1110, a first capacitive structure 1120, and a second capacitive structure 1130. As illustrated in FIG. 11, the first capacitive structure 1120 and the second capacitive structure 1130 are distributed on two sides of the flexible substrate 1110 along a thickness direction, and a projection of the first capacitive structure 1120 on the flexible substrate 1110 at least partially overlaps with a projection of the second capacitive structure 1130 on the flexible substrate 1110. In some embodiments, as illustrated in FIG. 12, the first capacitive structure 1120 and the second capacitive structure 1130 are distributed on a same side of the flexible substrate 1110 along the thickness direction.

The flexible substrate 1110 has flexible characteristics and tends to deform (e.g., bend) in response to an external force. In some embodiments, in order to fit the finger joint more easily, the flexible substrate 1110 may have a flat structure. In this case, the flexible substrate 1110 has a thickness direction, which may correspond to the Z-axis direction as illustrated in FIG. 11.

The capacitive structure (including the first capacitive structure 1120 and the second capacitive structure 1130) is a structure configured to measure bending deformation of the flexible substrate 1110. Specifically, the capacitive structure has a corresponding area, and electrical quantity (e.g., capacitance) of the capacitive structure is proportional to its area (or dimensional parameters affecting the area, such as the length, width of the capacitive structures, or a bending angle of the second strain sensor). In other words, electrical quantities of the capacitive structure in different directions are proportional to deformation amounts of the second strain sensor in different degrees of freedom, thereby reflecting a deformation condition (e.g., bending angles, bending directions). It should be understood that the second strain sensor may include, but is not limited to, capacitive structures; for example, it may also include resistive structures or capacitive-resistive composite structures. In some embodiments, each of the first capacitive structure 1120 and the second capacitive structure 1130 may include a multilayer structure disposed on a same side surface of the flexible substrate 1110 along a thickness direction (e.g., the Z-axis direction as illustrated in FIG. 11 or FIG. 12), and each layer of the multilayer structure is stacked along the thickness direction. The multilayer structure refers to a structure formed by stacking layered structures. The stacking of the layers of each multilayer structure along the thickness direction may be understood as follows: the layers in the multiple layered structures of the first capacitive structure 1120 and the second capacitive structure 1130 are arranged along the thickness direction of the flexible substrate 1110 and are stacked together. In some embodiments, the multilayer structure includes a second conductive layer, an intermediate layer, and a first conductive layer. In some embodiments, the multilayer structure includes a second conductive layer, a second intermediate layer, a third conductive layer, a first intermediate layer, and a first conductive layer. Specific layered structures included in the multilayer structure may be found in the following descriptions.

When different positions of the second strain sensor 1100 are respectively provided with different capacitive structures, these capacitive structures exhibit differentiated responses to deformations of the flexible substrate 1110 in different dimensions. When the flexible substrate 1110 undergoes a deformation in a certain dimension, the detection parameters generated by the capacitive structures exhibit characteristics corresponding to the deformation in that dimension; whereas when the flexible substrate 1110 undergoes a deformation in another dimension, the detection parameters generated by the capacitive structures exhibit characteristics corresponding to that dimension. It can be understood that the characteristics of the detection parameters generated by the capacitive structures correspond one-to-one with the deformation dimensions of the flexible substrate 1110. On this basis, the deformation of the flexible substrate 1110 may be identified based on the detection parameters of each of the capacitive structures. In some embodiments, a processor (e.g., the microprocessor 120 or the external computing device) may determine deformation of the flexible substrate 1110 in at least two dimensions based on capacitance-related parameters of each capacitive structure (e.g., the first capacitive structure 1120 and the second capacitive structure 1130) using a specific algorithm. For example, the deformation of the flexible substrate 1110 in at least two dimensions may be determined based on a machine learning model, a mapping relationship, or a functional relationship. More descriptions regarding the deformation in at least two dimensions may be found in other contents of the present disclosure (e.g., descriptions in connection with the following contents).

In some embodiments, when the second strain sensor 1100 (the flexible substrate 1110) undergoes bending deformation, at least a portion of the capacitive structures (e.g., the first capacitive structure 1120 and the second capacitive structure 1130) located on the flexible substrate 1110 may experience corresponding physical shape changes, thereby causing variations in the corresponding capacitance. For example, the bending of the second strain sensor 1100 may lead to a change in the areas of the capacitive structures, thereby altering the capacitance of the capacitive structures. By acquiring the capacitance of the capacitive structures or parameters related to the capacitance, and analyzing the variations in the capacitance or the capacitance-related parameters of the capacitive structures, the bending condition (e.g., bending angle, bending direction) of the second strain sensor 1100 may be accurately sensed.

As illustrated in FIG. 11, in some embodiments, the first capacitive structure 1120 and the second capacitive structure 1130 are respectively disposed on two sides of the flexible substrate 1110 along a thickness direction of the flexible substrate 1110. In some embodiments, the first capacitive structure 1120 and the second capacitive structure 1130 are symmetrically arranged with respect to the flexible substrate 1110 along the thickness direction. In this case, a projection of the first capacitive structure 1120 on the flexible substrate 1110 completely overlaps with a projection of the second capacitive structure 1130 on the flexible substrate 1110. When the first capacitive structure 1120 and the second capacitive structure 1130 are symmetrically arranged along the thickness direction with respect to the flexible substrate 1110, the two capacitive structures have the same response to external interferences (e.g., overall stretching or compression of the second strain sensor along a certain direction). In such cases, based on capacitance-related parameters of the capacitive structures, external interference on the second strain sensor 1100 may be eliminated using a differential processing algorithm, thereby improving sensitivity in determining deformation of the flexible substrate 1110 in at least two degrees of freedom.

In some embodiments, the flexible substrate 1110 includes a short-axis direction and a long-axis direction perpendicular to the thickness direction. The deformation of the second strain sensor 1100 (i.e., the flexible substrate 1110) illustrated in FIG. 11 in a plurality of degrees of freedom at least includes bending deformation around an axis parallel to the short-axis direction and tensile or compressive deformation along the long-axis direction. The short-axis direction may correspond to the X-axis direction illustrated in FIG. 11, and the long-axis direction may correspond to the Y-axis direction illustrated in FIG. 11.

As illustrated in FIG. 11, when the second strain sensor 1100 undergoing bending deformation around an axis parallel to the short-axis direction (i.e., the X-axis direction), the first capacitive structure 1120 and the second capacitive structure 1130 are respectively subjected to tensile deformation and compressive deformation. For example, the first capacitive structure 1120 is bent and elongated, and the second capacitive structure 1130 is bent and compressed. In this case, the capacitances of the first capacitive structure 1120 and the second capacitive structure 1130 change in opposite directions. Under such circumstances, a difference between the capacitances of the first capacitive structure 1120 and the second capacitive structure 1130 may reflect a bending direction and a bending magnitude of the second strain sensor 1100 around the axis parallel to the X-axis direction. Since the degrees to which the first capacitive structure 1120 and the second capacitive structure 1130 are stretched or compressed are substantially the same, a sum of their capacitances may remain substantially unchanged.

When the second strain sensor 1100 undergoing tensile or compressive deformation along a long-axis direction, the first capacitive structure 1120 and the second capacitive structure 1130 are synchronously stretched or compressed. In this case, the capacitances of the first capacitive structure 1120 and the second capacitive structure 1130 change synchronously. Under such circumstances, a difference between the capacitances of the first capacitive structure 1120 and the second capacitive structure 1130 remains substantially unchanged (or approximately zero), while a sum of the two capacitances may reflect a degree of stretching or compression of the second strain sensor 1100 along the long-axis direction.

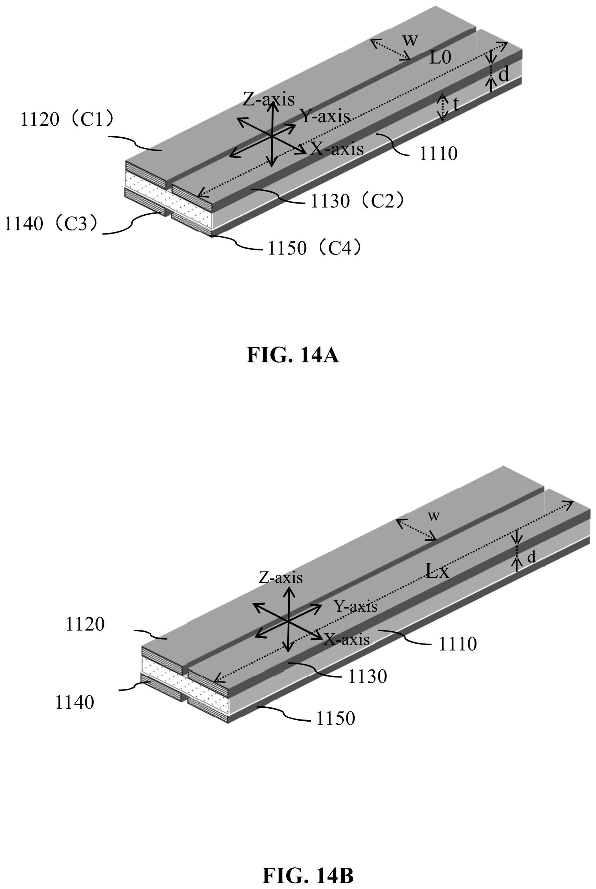

In some embodiments, by comparing characteristics of capacitances of the first capacitive structure 1120 and the second capacitive structure 1130 in the two degrees of freedom, e.g., a relationship between a sum or a difference of the capacitances and the deformation of the flexible substrate 1110, interference between deformations in the two degrees of freedom may be avoided, thereby effectively distinguishing deformation of the flexible substrate 1110 in the two degrees of freedom. For example, when the second strain sensor 1100 is stretched or compressed as a whole, the first capacitive structure 1120 and the second capacitive structure 1130 are synchronously stretched or compressed. Such synchronous stretching or compression may be regarded as common-mode interference, and a differential processing of the signals (e.g., capacitances) may eliminate such common-mode interference, such that the second strain sensor 1100 becomes insensitive to its own stretching or compression and only sensitive to bending deformation around an axis parallel to the short-axis direction (i.e., the X-axis direction), thereby enabling the second strain sensor 1100 to accurately detect bending deformation in this degree of freedom. In other words, a difference between the capacitances of the first capacitive structure 1120 and the second capacitive structure 1130 mainly reflects the bending deformation of the flexible substrate 1110 around an axis parallel to the short-axis direction (i.e., the X-axis direction). Similarly, a sum of the capacitances of the first capacitive structure 1120 and the second capacitive structure 1130 mainly reflects the stretching or compression deformation of the flexible substrate 1110 along the long-axis direction. More descriptions regarding a detection principle of the stretching or compression deformation in the long-axis direction and a detection principle of the second strain sensor 1100 having four capacitive structures may be found in other contents of the present disclosure (e.g., descriptions in connection with FIGs. 14A to 16).

As illustrated in FIG. 12, in some embodiments, the flexible substrate 1110 includes a long-axis direction perpendicular to a thickness direction, the first capacitive structure 1120 and the second capacitive structure 1130 are disposed on a same side of the flexible substrate 1110 in the thickness direction, the first capacitive structure 1120 and the second capacitive structure 1130 are arranged side by side, and both extend along the long-axis direction of the flexible substrate 1110.

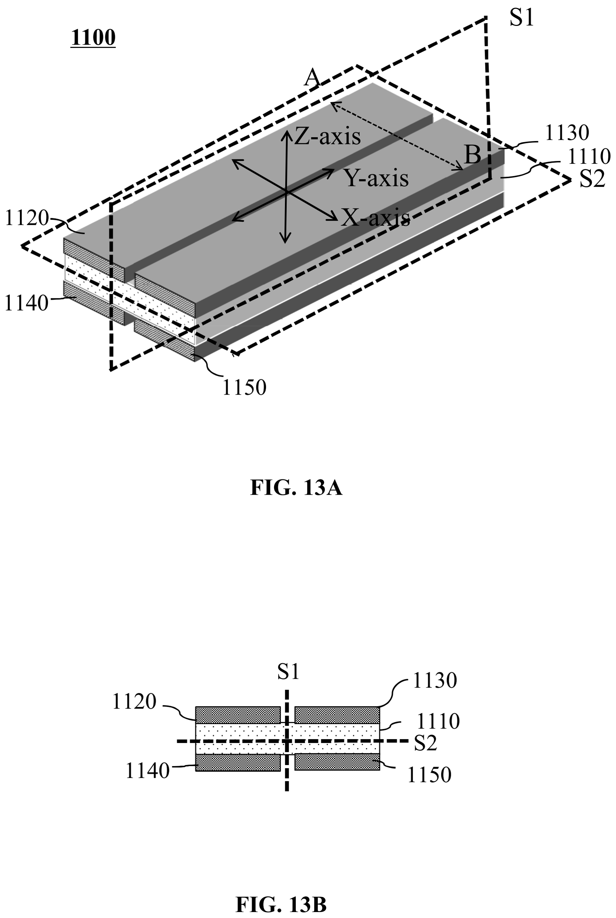

In some embodiments, the first capacitive structure 1120 and the second capacitive structure 1130 are symmetrically disposed with respect to a mid-section parallel to a plane formed by the thickness direction and the long-axis direction, the mid-section being located on the flexible substrate 1110. When the first capacitive structure 1120 and the second capacitive structure 1130 is symmetrically disposed on the mid-section with respect to the flexible substrate 1110, the two capacitive structures exhibit the same response to a portion of external interferences (e.g., a uniform stretching or compression of the second strain sensor 1100 along a certain direction). In this case, the processor may eliminate an influence of the external interference on the second strain sensor 1100 based on capacitance-related parameters of the capacitive structures (e.g., the first capacitive structure 1120 and the second capacitive structure 1130) using a differential processing algorithm, thereby improving a sensitivity of determining deformation of the flexible substrate 1110 in at least two degrees of freedom. More descriptions regarding the mid-section may be found in other contents of the present disclosure (e.g., descriptions in connection with FIGs. 13A and 13B).

In some embodiments, degrees of freedom of deformation generated by the second strain sensor 1100 (the flexible substrate 1110) illustrated in FIG. 12 at least include a bending deformation around an axis parallel to a thickness direction and a stretching or compression deformation along a long-axis direction.

As illustrated in FIG. 12, when the second strain sensor 1100 undergoing a bending deformation around an axis parallel to the thickness direction (i.e., a Z-axis direction), the first capacitive structure 1120 and the second capacitive structure 1130 respectively undergo a stretching deformation and a compression deformation. For example, the first capacitive structure 1120 is bent and elongated, and the second capacitive structure 1130 is bent and compressed. In this case, capacitances of the first capacitive structure 1120 and the second capacitive structure 1130 change in opposite directions. In this case, a difference in capacitances between the first capacitive structure 1120 and the second capacitive structure 1130 may reflect a bending direction and a bending degree of the second strain sensor 1100 around an axis parallel to the Z-axis direction, and a sum of capacitances of the first capacitive structure 1120 and the second capacitive structure 1130 may remain substantially unchanged due to a substantially same degree of stretching and compression experienced by the first capacitive structure 1120 and the second capacitive structure 1130.

When the second strain sensor 1100 illustrated in FIG. 12 undergoes stretching or compression deformation along the long-axis direction, the first capacitive structure 1120 and the second capacitive structure 1130 may be synchronously stretched or compressed, resulting in synchronous changes in capacitances of the first capacitive structure 1120 and the second capacitive structure 1130. In this case, a difference in capacitances between the first capacitive structure 1120 and the second capacitive structure 1130 may remain substantially unchanged (or close to zero), while a sum of capacitances of the first capacitive structure 1120 and the second capacitive structure 1130 may reflect a degree of stretching or compression of the second strain sensor 1100 along the long-axis direction.

In some embodiments, by comparing characteristics of capacitance of the first capacitive structure 1120 and the second capacitive structure 1130 under the two aforementioned degrees of freedom, for example, a relationship between a sum (or difference) of capacitances and a deformation of the flexible substrate 1110, interference between the deformations of the two degrees of freedom may be avoided, thereby effectively distinguishing the deformations of the flexible substrate 1110 in the two degrees of freedom. For example, when the second strain sensor 1100 undergoes overall stretching or compression, the first capacitive structure 1120 and the second capacitive structure 1130 are synchronously stretched or compressed. The synchronous stretching or compression of the first capacitive structure 1120 and the second capacitive structure 1130 may be regarded as common-mode interference. In this case, differential processing may be performed on signals (e.g., capacitance) to eliminate the common-mode interference, such that the second strain sensor 1100 is insensitive to stretching or compression deformation of the second strain sensor 1100 along the long-axis direction, and is only sensitive to bending deformation around an axis parallel to the thickness direction (i.e., Z-axis direction). Therefore, the second strain sensor 1100 is enabled to accurately detect the bending deformation corresponding to the degree of freedom around the thickness direction. A difference in capacitances between the first capacitive structure 1120 and the second capacitive structure 1130 mainly reflects a bending deformation of the flexible substrate 1110 around an axis parallel to the thickness direction (i.e., the Z-axis direction). Similarly, a sum of capacitances of the first capacitive structure 1120 and the second capacitive structure 1130 mainly reflects a stretching or compression deformation of the flexible substrate 1110 along a long-axis direction. More descriptions regarding a detection principle for stretching or compression deformation along the long-axis direction, which is similar to a detection principle of the second strain sensor 1100 having four capacitive structures, may be found in other contents of the present disclosure (e.g., descriptions in connection with FIGs. 14A to 16).

FIG. 13A is a schematic diagram illustrating a distribution of four capacitive structures on a flexible substrate according to some embodiments of the present disclosure; FIG. 13B is a schematic diagram illustrating a cross-sectional view of the four capacitive structures shown in FIG. 13A along a viewing direction of the Y-axis.

As illustrated in FIGs. 13A and 13B, in some embodiments, compared to FIG. 12, the second strain sensor 1100 further includes a third capacitive structure 1140 and a fourth capacitive structure 1150. The first capacitive structure 1120 and the second capacitive structure 1130 are arranged side by side on one side of the flexible substrate 1110 along a thickness direction and both extend along a long-axis direction; the third capacitive structure 1140 and the fourth capacitive structure 1150 are arranged side by side on another side of the flexible substrate 1110 along the thickness direction and both extend along the long-axis direction.

In some embodiments, the processor is configured to read parameters related to capacitances of the first capacitive structure 1120, the second capacitive structure 1130, the third capacitive structure 1140, and the fourth capacitive structure 1150, and determine deformations of the flexible substrate 1110 in at least two degrees of freedom based on these parameters.

In some embodiments, degrees of freedom of deformation generated by the second strain sensor 1100 (the flexible substrate 1110) illustrated in FIG. 13A at least include bending deformation around an axis parallel to a thickness direction, bending deformation around an axis parallel to a short-axis direction, and tensile or compressive deformation along a long-axis direction of the flexible substrate 1110. For illustrative purposes, descriptions of the parameters related to capacitance of the respective capacitive structures under deformations in different degrees of freedom are provided below in connection with FIGs. 14A, 14B, 15, and 16.

In some embodiments, the parameters related to capacitance include: C1, C2, C3, C4, Ctotal, dCx, and dCz; where $Ctotal = C1 + C2 + C3 + C4$, the composite differential value $dCx = (C1 + C2) - (C3 + C4)$, and the composite differential value $dCz = (C1 + C3) - (C2 + C4)$. C1, C2, C3, and C4 respectively represent the capacitances of the first capacitive structure 1120, the second capacitive structure 1130, the third capacitive structure 1140, and the fourth capacitive structure 1150.

In some embodiments, the first capacitive structure 1120 and the second capacitive structure 1130 are symmetrically arranged with respect to a first central section S1, and the third capacitive structure 1140 and the fourth capacitive structure 1150 are also symmetrically arranged with respect to the first central section S1. The first central section S1 refers to a section of the flexible substrate 1110 that is parallel to a plane formed by the thickness direction and the long-axis direction. The first capacitive structure 1120 and the third capacitive structure 1140 are symmetrically arranged with respect to a second central section S2 of the flexible substrate 1110, and the second capacitive structure 1130 and the fourth capacitive structure 1150 are also symmetrically arranged with respect to the second central section S2. The second central section S2 refers to a central surface of the flexible substrate 1110 that is parallel to a plane formed by the long-axis direction and the short-axis direction.

By arranging four symmetric capacitive structures on the second strain sensor 1100 and performing composite differential operations, common-mode interference may be effectively eliminated during the process of identifying bending deformation of the sensor. Merely by way of example, when stretching or compressing deformation of the second strain sensor 1100 as a whole, the first capacitive structure 1120, the second capacitive structure 1130, the third capacitive structure 1140, and the fourth capacitive structure 1150 are synchronously stretched or compressed. The synchronous stretching or compression of the four capacitive structures is regarded as common-mode interference. The parameters dCx and dCz obtained by performing composite differential processing on signals from the four capacitive structures remain unchanged, which effectively eliminates such common-mode interference, such that the flexible second strain sensor 1100 is insensitive to its own stretching or compressing deformation and only sensitive to its own bending deformation, thereby improving accuracy of the second strain sensor 1100.

FIG. 14A is a schematic diagram illustrating an undeformed state of a second sensor according to some embodiments of the present disclosure; FIG. 14B is a schematic diagram illustrating deformation of the second sensor shown in FIG. 14A after being stretched or compressed along a long axis.

In some embodiments, when each of the first capacitive structure 1120, the second capacitive structure 1130, the third capacitive structure 1140, and the fourth capacitive structure 1150 is a capacitive structure, the second strain sensor 1100 is capable of identifying stretching or compressing deformation along a long-axis direction of the flexible substrate 1110 based on parameters related to capacitance.

In some embodiments, as illustrated in FIG. 14A, when the second strain sensor 1100 is not subjected to deformation, capacitances of the four capacitive structures may be determined by the following Equation (1). For illustration purposes, the four capacitive structures of the second strain sensor 1100 are regarded as completely identical and symmetrically arranged with respect to the flexible substrate 1110 (i.e., the first central section S1 and the second central section S2).

$$C1 = C2 = C3 = C4 = \varepsilon_0 \varepsilon \frac{A}{d} = \varepsilon_0 \varepsilon \frac{L0w}{d} \qquad (1)$$

where $\varepsilon_0$ denotes a vacuum permittivity constant; $\varepsilon$ denotes respective relative permittivities of the four capacitive structures (i.e., intermediate layers, see descriptions provided below); d denotes respective thicknesses of dielectric layers of the four capacitive structures; A denotes respective areas of the four capacitive structures in a plane defined by the long-axis direction and the short axis direction; L0 denotes respective initial lengths of the four capacitive structures in the long-axis direction of the flexible substrate 1110; and w denotes respective widths of the four capacitive structures in the short-axis direction of the flexible substrate 1110.

In some embodiments, as illustrated in FIG. 14B, when the second strain sensor 1100 is stretched or compressed along the long-axis direction (i.e., the Y-axis direction), the respective initial length $L_o$ of each of the four capacitive structures is stretched or compressed to Lx. Considering that the thickness variation of each of the four capacitive structures is minimal, for convenience of explanation, the thickness of each of the four capacitive structures is approximately regarded as unchanged. Then, based on Equation (1), the capacitance variations of the four capacitive structures are expressed by the following Equation (2).

$$C1 = C2 = C3 = C4 = \varepsilon_0 \varepsilon \frac{Lxw}{d}. \qquad (2)$$

Ctotal, dCx, dCz are respectively determined by the following Equations (3), (4), and (5).

$$Ctotal = C1 + C2 + C3 + C4 = 4\varepsilon_0 \varepsilon \frac{Lxw}{d} \qquad (3)$$

$$dCx = (C1 + C2) - (C3 + C4) = 0 \qquad (4)$$

$$dCz = (C1 + C3) - (C2 + C4) = 0. \qquad (5)$$

When the second strain sensor 1100 undergoes tensile or compressive deformation along the long-axis direction, the four capacitive structures are stretched or compressed synchronously, and capacitance values of the four capacitive structures change in a synchronized manner. In this case, a sum of capacitance values of the four capacitive structures may reflect a degree of tensile or compressive deformation of the second strain sensor 1100 (flexible substrate 1110) along the long-axis direction. According to Equations (2) and (3), Ctotal is only related to the length Lx of the capacitive structures after deformation, and Lx may be used to indicate the tensile or compressive deformation of the second strain sensor 1100 along the long-axis direction. Therefore, Ctotal may be used to reflect the tensile or compressive deformation of the second strain sensor 1100 (flexible substrate 1110) along the long-axis direction.

According to Equation (4), dCx reflects a difference between a sum of capacitance C1 of the first capacitive structure 1120 and capacitance C2 of the second capacitive structure 1130, and a sum of capacitance C3 of the third capacitive structure 1140 and capacitance C4 of the fourth capacitive structure 1150. Referring to the structural distribution of the four capacitive structures in FIG. 14B, a sum of capacitance C1 of the first capacitive structure 1120 and capacitance C2 of the second capacitive structure 1130, i.e., C1 + C2, may reflect a bending deformation of an upper side surface of the flexible substrate 1110 along a thickness direction. A sum of capacitance C3 of the third capacitive structure 1140 and capacitance C4 of the fourth capacitive structure 1150, i.e., C3 + C4, may reflect a bending deformation of a lower side surface of the flexible substrate 1110 along the thickness direction. Therefore, dCx is configured to reflect a difference in bending deformations between the upper and lower side surfaces (i.e., upper and lower surfaces along the thickness direction) of the flexible substrate 1110 during deformation, which is related to a bending deformation about an axis parallel to the short-axis direction (i.e., the X-axis direction). A stretching or compressing deformation along the long-axis direction (i.e., the Y-axis direction) does not cause a difference in deformations between the upper and lower side surfaces of the flexible substrate 1110, so dCx is a constant value of 0.

Similarly, according to Equation (5), dCz is configured to reflect a difference in bending deformations between two side surfaces of the flexible substrate 1110 along the short-axis direction during deformation. dCz is only related to a bending deformation about an axis parallel to the thickness direction (i.e., the Z-axis direction). A stretching or compressing deformation along the long-axis direction (i.e., the Y-axis direction) does not cause a difference in deformations between the two side surfaces of the flexible substrate 1110 along the short-axis direction, so dCz is also a constant value of 0.

In some embodiments, an output parameter Ctotal of the second strain sensor 1100 under stretching or compressing along the Y-axis changes linearly and proportionally with a stretching length or compressing length Lx, exhibiting extremely high linearity, whereas both dCz and dCx remain 0 and are not affected by the stretching length or compressing length Lx. Therefore, the second strain sensor 1100 is capable of identifying a stretching or compressing deformation along a long-axis direction of the flexible substrate 1110 based on a capacitance-related parameter Ctotal.

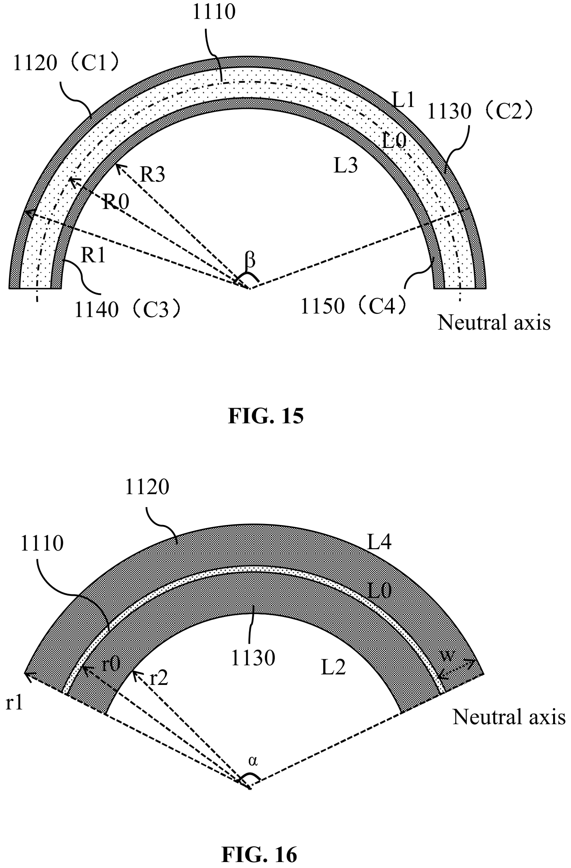

FIG. 15 is a schematic diagram illustrating a side view of the second sensor shown in FIG. 14A after being bent around an axis parallel to a short axis direction.

In some embodiments, when the first capacitive structure 1120, the second capacitive structure 1130, the third capacitive structure 1140, and the fourth capacitive structure 1150 are all capacitive structures, the second strain sensor 1100 is capable of identifying a bending deformation of the flexible substrate 1110 around an axis parallel to a short-axis direction based on a capacitance-related parameter dCx.

As illustrated in FIGs. 14A and 15, when the second strain sensor 1100 undergoes a bending deformation around an axis parallel to a short-axis direction (i.e., the X-axis direction), the deformed second strain sensor 1100 may be approximated as an arc for illustrative purposes. The first capacitive structure 1120 and the second capacitive structure 1130 are bent and elongated to L1, while the third capacitive structure 1140 and the fourth capacitive structure 1150 are bent and compressed to L3. Considering that a thickness of each of the four capacitive structures changes very slightly, the thicknesses of the four capacitive structures are approximately assumed to remain constant for ease of explanation. In this case, based on Equation (1), changes in the capacitance C1 of the first capacitive structure 1120 and the capacitance C2 of the second capacitive structure 1130 are expressed by Equation (6), and changes in the capacitance C3 of the third capacitive structure 1140 and the capacitance C4 of the fourth capacitive structure 1150 are expressed by Equation (7).

$$C1 = C2 = \varepsilon_0 \varepsilon \frac{L1w}{d} = \varepsilon_0 \varepsilon \frac{R1\beta w}{d} = \varepsilon_0 \varepsilon \frac{(R0+t/2)\beta w}{d} \qquad (6)$$

$$C3 = C4 = \varepsilon_0 \varepsilon \frac{L3w}{d} = \varepsilon_0 \varepsilon \frac{R3\beta w}{d} = \varepsilon_0 \varepsilon \frac{(R0-t/2)\beta w}{d}. \qquad (7)$$

In this case, Ctotal, dCx, dCz are respectively determined by the following Equations (8), (9), and (10).

$$Ctotal = C1 + C2 + C3 + C4 = 4\varepsilon_0 \varepsilon \frac{L0w}{d} \qquad (8)$$

$$dCx = (C1 + C2) - (C3 + C4) = \frac{2\varepsilon_0 \varepsilon wt}{d}\beta \qquad (9)$$

$$dCz = (C1 + C3) - (C2 + C4) = 0 \qquad (10)$$

where R0 denotes a radius of the arc of the bending deformation of the flexible substrate 1110; R1 denotes a radius of the arc of the bending deformation of the first capacitive structure 1120 and the second capacitive structure 1130; R2 denotes a radius of the arc of the bending deformation of the third capacitive structure 1140 and the fourth capacitive structure 1150; t denotes a thickness of the second strain sensor 1100 as illustrated in FIG. 14A; L1 denotes an arc length of the bending deformation of the first capacitive structure 1120 and the second capacitive structure 1130; and L3 denotes an arc length of the bending deformation of the third capacitive structure 1140 and the fourth capacitive structure 1150.

When the second strain sensor 1100 undergoes a bending deformation along an axis parallel to the short axis direction, for example, the first capacitive structure 1120 and the second capacitive structure 1130 are bent and stretched, and the third capacitive structure 1140 and the fourth capacitive structure 1150 are bent and compressed, a sum of capacitances C1 and C2 of the first capacitive structure 1120 and the second capacitive structure 1130, i.e., C1+C2, and a sum of capacitances C3 and C4 of the third capacitive structure 1140 and the fourth capacitive structure 1150, i.e., C3+C4, exhibit opposite variation trends. In this case, a total capacitance Ctotal obtained by adding the capacitances of the four capacitive structures can offset the variation trend of C1+C2 and C3+C4, and Ctotal is a constant as shown in Equation (8). Therefore, Ctotal is not suitable for reflecting the bending deformation of the second strain sensor 1100 along an axis parallel to the short axis direction.

In some embodiments, the output parameter dCx of the second strain sensor 1100 after bending deformation along an axis parallel to the short-axis direction (i.e., the X-axis direction) varies linearly and proportionally with the bending angle β, exhibiting extremely high linearity, whereas dCz and Ctotal remain constant and are not affected by the bending angle β. Therefore, the second strain sensor 1100 is capable of identifying bending deformation around an axis parallel to the short-axis direction based on the capacitance-related parameter dCx.

It should be noted that, in addition to the case described above with four capacitive structures, the second strain sensor 1100 may include three or more capacitive structures distributed on both side surfaces of the flexible substrate 1110 along the thickness direction and extending along the long-axis direction, as long as deformation in different dimensions can be characterized by capacitance-related parameters of these capacitive structures to identify multidimensional deformation. Merely by way of example, the second strain sensor 1100 may include three capacitive structures, two of which are distributed on an upper side surface of the flexible substrate and the other one is distributed on a lower side surface of the flexible substrate. In this case, the bending deformation of the second strain sensor 1100 around the X-axis or the Z-axis may also be determined based on the capacitance-related parameters respectively generated by the three capacitive structures.

FIG. 16 is a schematic diagram illustrating a top view of the second sensor shown in FIG. 14A after being bent around an axis parallel to a thickness direction.

In some embodiments, when the first capacitive structure 1120, the second capacitive structure 1130, the third capacitive structure 1140, and the fourth capacitive structure 1150 are all capacitive structures, the second strain sensor 1100 is capable of identifying the bending deformation of the flexible substrate 1110 around an axis parallel to the thickness direction based on the capacitance-related parameter dCz.

As illustrated in FIGs. 14A and 16, in some embodiments, after the second strain sensor 1100 undergoes bending deformation along an axis parallel to the thickness direction (i.e., the Z-axis direction), the second strain sensor 1100 is approximately regarded as forming an arc for ease of explanation. The first capacitive structure 1120 and the third capacitive structure 1140 (not shown in FIG. 16) are bent and elongated to L4, and the second capacitive structure 1130 and the fourth capacitive structure 1150 (not shown in FIG. 16) are bent and compressed to L2. Considering that a thickness change of each of the four capacitive structures is minimal, the thicknesses of the four capacitive structures are approximately regarded as unchanged for ease of explanation. In this case, based on Equation (1), the capacitance C1 of the first capacitive structure 1120 and the capacitance C3 of the third capacitive structure 1140 vary according to Equation (11), and the capacitance C2 of the second capacitive structure 1130 and the capacitance C4 of the fourth capacitive structure 1150 vary according to Equation (12).

$$C1 = C3 = \varepsilon_0 \varepsilon \frac{(L0+L4)\ w}{2d} = \varepsilon_0 \varepsilon \frac{(r0\alpha + r1\alpha)w}{2d} = \varepsilon_0 \varepsilon \frac{(2r0+w)\alpha w}{2d} \qquad (11)$$

$$C2 = C4 = \varepsilon_0 \varepsilon \frac{(L0+L2)\ w}{2d} = \varepsilon_0 \varepsilon \frac{(r0\alpha + r2\alpha)w}{2d} = \varepsilon_0 \varepsilon \frac{(2r0-w)\alpha w}{2d}. \qquad (12)$$

Ctotal, dCx, dCz are respectively determined by the following Equations (13), (14), and (15).

$$Ctotal = C1 + C2 + C3 + C4 = 4\varepsilon_0 \varepsilon \frac{L0w}{d} \qquad (13)$$

$$dCx = (C1 + C2) - (C3 + C4) = 0 \qquad (14)$$

$$dCz = (C1 + C3) - (C2 + C4) = \frac{2\varepsilon_0 \varepsilon w^2}{d}\alpha, \qquad (15)$$

where r0 denotes a radius of an arc corresponding to a bending deformation of the flexible substrate 1110; r1 denotes a radius of an arc corresponding to the bending deformation of the first capacitive structure 1120 and the third capacitive structure 1140; r2 denotes a radius of an arc corresponding to the bending deformation of the second capacitive structure 1130 and the fourth capacitive structure 1150; L2 denotes an arc length of the bending deformation of the first capacitive structure 1120 and the third capacitive structure 1140; L4 denotes an arc length of the bending deformation of the second capacitive structure 1130 and the fourth capacitive structure 1150.

In some embodiments, an output parameter dCz of the second strain sensor 1100 after undergoing a bending deformation around an axis parallel to the thickness direction (i.e., the Z-axis direction) varies linearly and proportionally with a bending angle $\alpha$, exhibiting extremely high linearity, whereas dCx and Ctotal are both constants and are not affected by the bending angle $\alpha$. Therefore, the second strain sensor 1100 is capable of identifying a bending deformation around an axis parallel to the thickness direction based on a capacitance-related parameter dCz.

In some embodiments, when the first capacitive structure 1120, the second capacitive structure 1130, the third capacitive structure 1140, and the fourth capacitive structure 1150 are all configured as resistive structures, the processor may be configured to determine deformation of the flexible substrate 1110 in at least two dimensions based on resistance-related parameters of the four resistive structures. For example, the resistances of the four capacitive structures are

respectively: $R1 = R2 = R3 = R4 = \rho \dfrac{d}{A}$. By substituting the area of each capacitive structure after deformation of the second strain sensor 1100 into the above equation and performing differential operations, results similar to those of capacitance-related parameters may be obtained, enabling the processor to determine the bending deformation of the second strain sensor 1100 about an axis parallel to the thickness direction, the bending deformation about an axis parallel to the short-axis direction, and the stretching or compression deformation along the long-axis direction of the flexible substrate. Wherein, $\rho$ denotes the resistivity of each of the four capacitive structures (i.e., the intermediate layers, see the following descriptions).

In some embodiments, the processor is configured to identify a composite deformation in which any combination of the following deformations coexist: bending deformation of the second strain sensor 1100 about an axis parallel to the thickness direction, bending deformation about an axis parallel to the short-axis direction, and tensile or compressive deformation along the long-axis direction of the flexible substrate 1110. For example, the processor is configured to simultaneously calculate variations in the capacitance-related parameters Ctotal, dCx, and dCz, thereby restoring deformation components in different dimensions concurrently, so as to reconstruct the actual bending deformation of the second strain sensor 1100.

Accordingly, the embodiments of the present disclosure enable deformation in multiple degrees of freedom (e.g., bending around the X-axis, bending around the Z-axis, and stretching along the Y-axis) to be measured using a single sensor, thereby realizing detection of posture data in multiple dimensions of a finger, which is beneficial for improving detection accuracy while simplifying the fabrication process and facilitating miniaturized design.

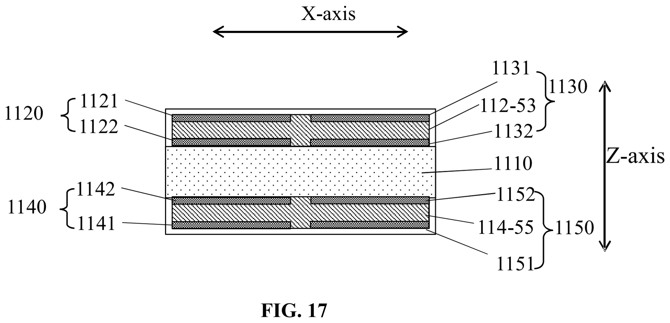

FIG. 17 is a schematic diagram illustrating a cross-sectional view of a second strain sensor according to some embodiments of the present disclosure.

As illustrated in FIG. 17, in some embodiments, each capacitive structure includes a second conductive layer, an intermediate layer, and a first conductive layer that are sequentially stacked in a direction away from the flexible substrate 1110 along a thickness direction. For example, the first capacitive structure 1120 includes a second conductive layer 1122, an intermediate layer 112-53, and a first conductive layer 1121 that are sequentially stacked in a direction away from the flexible substrate 1110; the second capacitive structure 1130 includes a second conductive layer 1132, an intermediate layer 112-53, and a first conductive layer 1131 that are sequentially stacked in a direction away from the flexible substrate 1110; the third capacitive structure 1140 includes a second conductive layer 1142, an intermediate layer 114-55, and a first conductive layer 1141 that are sequentially stacked in a direction away from the flexible substrate 1110; and the fourth capacitive structure 1150 includes a second conductive layer 1152, an intermediate layer 114-55, and a first conductive layer 1151 that are sequentially stacked in a direction away from the flexible substrate 1110. In some embodiments, the intermediate layer is disposed between the first conductive layer and the second conductive layer.

In some embodiments, the first conductive layer and the second conductive layer of each capacitive structure include an elastic conductive material. The elastic conductive material enables the first conductive layer and the second conductive layer to be electrically conductive and to return to an initial shape when the external force disappears. In some embodiments, the elastic conductive material includes, but is not limited to, a conductive adhesive film, conductive ink, conductive polymer material, conductive gel, liquid metal, etc. In some embodiments, the first conductive layer and the second conductive layer include the conductive adhesive film, which is prepared by mixing conductive particles with a polymer material. In some embodiments, the polymer material includes, but is not limited to, silica gel, rubber, resin, etc. In some embodiments, the first conductive layer and the second conductive layer include the conductive ink, which is prepared by mixing conductive particles with ink material, and the conductive ink is capable of forming a conductive pattern through printing. In some embodiments, the first conductive layer and the second conductive layer include conductive polymer materials, conductive gel, liquid metal, or the like. In some embodiments, the conductive polymer material includes, but is not limited to, polypyrrole, poly(3,4-ethylenedioxythiophene)-polystyrene sulfonate (PEDOT:PSS, Poly(3,4-ethylenedioxythiophene) polystyrene sulfonate), or the like.

In some embodiments, the elastic conductive material includes an elastic material internally filled with conductive particles. The conductivity of the first conductive layer and the second conductive layer may be adjusted by controlling the density of the conductive particles filled in the elastic material. In some embodiments, the elastic material includes silicone, rubber, resin, polydimethylsiloxane (PDMS), polyurethane, styrene-butadiene-styrene (SBS), or the like. In some embodiments, the conductive particles include metal powder and carbon powder, such as carbon nanotubes, silver nanowires, carbon black, graphite powder, graphene, or the like.

In some embodiments, the intermediate layer includes an elastic insulating material. In this case, each capacitive structure is a capacitive structure. For example, the elastic insulating material includes silicone, rubber, polydimethylsiloxane (PDMS), thermoplastic polyurethane (TPU), or the like. In some embodiments, the intermediate layer includes a high-resistance elastic material, the resistivity of the intermediate layer is more than 1000 times greater than that of the first conductive layer and the second conductive layer, and in this case, each capacitive structure is a composite capacitor-resistor structure.

In some embodiments, a thickness of the first conductive layer and the second conductive layer of each capacitive structure is in a range from 1 to 100 $\mu$m. In some embodiments, a width of the first conductive layer and the second conductive layer of each capacitive structure is in a range from 0.5 to 10 mm. In some embodiments, a spacing between the first conductive layer 1121 of the first capacitive structure 1120 and the first conductive layer 1131 of the second capacitive structure 1130 is in a range from 0.02 to 2 mm. In some embodiments, a spacing between the second conductive layer 1122 of the first capacitive structure 1120 and the second conductive layer 1132 of the second capacitive structure 1130 is in a range from 0.02 to 2 mm. In some embodiments, a spacing between the first conductive layer 1141 of the third capacitive structure 1140 and the first conductive layer 1151 of the fourth capacitive structure 1150 is in a range from 0.02 to 2 mm. In some embodiments, a spacing between the second conductive layer 1142 of the third capacitive structure 1140 and the second conductive layer 1152 of the fourth capacitive structure 1150 is in a range from 0.02 to 2 mm.

In some embodiments, a conductivity of the first conductive layer is greater than a conductivity of the intermediate layer.

The conductivity is a parameter used to describe the ease of charge flow in a substance. Since the conductivity of the first conductive layer is greater than the conductivity of the second conductive layer, the first conductive layer has better electrical conductivity. Therefore, a resistance of the first conductive layer is smaller than a resistance of the second conductive layer. It should be noted that in the present disclosure, the resistance of a component refers to a resistance between two surfaces of the component spaced along a thickness direction of the flexible substrate 1110. The resistance of the first conductive layer refers to the resistance between two surfaces of the first conductive layer spaced along the thickness direction of the flexible substrate 1110, and the resistance of the second conductive layer may refer to the resistance between two surfaces of the second conductive layer spaced along the thickness direction of the flexible substrate 1110. In some embodiments, the conductivity of the first conductive layer may be more than 100 times the conductivity of the second conductive layer. In some embodiments, by setting a density of conductive particles filled in the elastic material of the first conductive layer to be greater than a density of conductive particles filled in the elastic material of the second conductive layer, the conductivity of the first conductive layer may be greater than that of the second conductive layer.

When the capacitive structures (e.g., the first capacitive structure 1120, the second capacitive structure 1130, the third capacitive structure 1140, and the fourth capacitive structure 1150) are capacitive-resistive composite capacitive structures or single capacitive structures, a resistance between two surfaces of the intermediate layer spaced along a thickness direction of the flexible substrate 1110 should not be too small, so as to facilitate subsequent measurement of the resistance and analysis of a variation value of the resistance. In some embodiments, the resistance between the two surfaces of the intermediate layer spaced along the thickness direction of the flexible substrate 1110 is greater than 0.8 M$\Omega$. For example, when the capacitive structures (e.g., the first capacitive structure 1120, the second capacitive structure 1130, the third capacitive structure 1140, and the fourth capacitive structure 1150) are capacitive-resistive composite capacitive structures, the resistance between the two surfaces of the intermediate layer spaced along the thickness direction of the flexible substrate 1110 may be in a range from 0.8 M$\Omega$ to 1150 G$\Omega$. As another example, in response to the capacitive structures (e.g., the first capacitive structure 1120, the second capacitive structure 1130, the third capacitive structure 1140, and the fourth capacitive structure 1150) are single capacitive structures, the resistance between the two surfaces of the intermediate layer spaced along the thickness direction of the flexible substrate 1110 may be greater, for instance, the resistance may be greater than 1150 G$\Omega$.

By setting a resistance between two surfaces of the intermediate layer spaced along a thickness direction of the flexible substrate 1110 to be greater than 0.8 M$\Omega$, the intermediate layer is able to conduct electricity while maintaining a sufficiently large resistance, thereby ensuring the measurement of the resistance of the intermediate layer and the analysis of the variation value of the resistance, which can accurately reflect a bending condition of the second strain sensor 1100.

In some embodiments, a relative permittivity of the intermediate layer is greater than 2. The relative permittivity refers to a physical parameter characterizing dielectric properties or polarization properties of a dielectric material, and is equal to a ratio of capacitance of a capacitor having the corresponding material as a dielectric to capacitance of a capacitor of the same size using vacuum as a dielectric. The value of the relative permittivity also represents the charge storage capacity of the material, and is also referred to as relative dielectric constant. A material with a relative permittivity greater than 2 is a polar material. In other words, an intermediate layer with a relative permittivity greater than 2 has a certain charge storage capacity. Therefore, the intermediate layer may be equivalent to a parallel combination of a resistive component and a capacitive component.

In some embodiments, the relative permittivity of the intermediate layer may be greater than or equal to 4. Preferably, the relative permittivity of the intermediate layer is greater than 5. In some embodiments, the relative permittivity of the intermediate layer is greater than 10.

By setting the relative permittivity of the intermediate layer to be greater than 2, the intermediate layer serves as both a resistive component and a capacitive component. During the bending process of the second strain sensor 1100, both the capacitance and resistance of the intermediate layer vary with the deformation of the flexible substrate 1110. Therefore, parameters related to the capacitance and resistance of the intermediate layer may reflect the bending state of the second strain sensor 1100. In the present embodiment, the simultaneous presence of capacitance and resistance for reflecting the bending state of the second strain sensor 1100 significantly improves the sensitivity and accuracy of the second strain sensor 1100.

Additionally, by setting the resistance between two surfaces of the intermediate layer spaced along the thickness direction of the flexible substrate 1110 to be greater than $0.8\,\text{M}\Omega$, the intermediate layer can exhibit both favorable resistive performance and favorable capacitive performance, thereby ensuring the sensitivity and accuracy of the second strain sensor 1100.

In some embodiments, two capacitive structures on the same surface of the flexible substrate 1110 may share a first conductive layer, and the second conductive layers of the two capacitive structures are spaced apart along the short-axis direction of the flexible substrate 1110. In some embodiments, additional conductive layers (e.g., third conductive layers) may be stacked in the thickness direction of each capacitive structure, which can effectively improve the sensitivity of the sensor 1100 in detecting deformation in each degree of freedom. For example, each capacitive structure includes, in the thickness direction and sequentially away from the flexible substrate 1110, a second conductive layer, a second intermediate layer, a third conductive layer, a first intermediate layer, and a first conductive layer. The third conductive layer, the first conductive layer, and the second conductive layer have the same parameters; the second intermediate layer, the first intermediate layer, and the intermediate layer described above have the same parameters.

In some embodiments of the present disclosure, by applying a stopping structure (e.g., a ratchet, or the like) in cooperation with a stopping member to the finger force feedback component to perform force feedback, the structure of the finger force feedback component can be simplified, and the size of the finger force feedback component can be reduced, thereby enabling the miniaturization of the force feedback glove while reducing power consumption.

The foregoing has described the basic concepts. Clearly, for those skilled in the art, the above detailed disclosure is merely provided as an example and does not constitute a limitation of the present disclosure. Although not explicitly stated herein, those skilled in the art may make various modifications, improvements, and adaptations to the present disclosure. Such modifications, improvements, and adaptations are suggested in the present disclosure and therefore fall within the spirit and scope of the exemplary embodiments of the present disclosure.

Meanwhile, the present disclosure uses specific terms to describe embodiments of the present disclosure. Expressions such as "one embodiment," "an embodiment," and/or "some embodiments" refer to a certain feature, structure, or characteristic associated with at least one embodiment of the present disclosure. Therefore, it should be emphasized and noted that the repeated references to "an embodiment," "one embodiment," or "an alternative embodiment" in different parts of the present disclosure do not necessarily refer to the same embodiment. In addition, certain features, structures, or characteristics in one or more embodiments of the present disclosure may be appropriately combined. Furthermore, unless explicitly stated in the claims, the order of processing elements and sequences, the use of numerals or letters, or the use of other names in the present disclosure are not intended to limit the sequence of processes and methods described herein.

Furthermore, unless explicitly stated in the claims, the order of processing elements and sequences, the use of numerals or letters, or the use of other names in the present disclosure are not intended to limit the sequence of processes and methods described herein. Although some presently considered useful embodiments have been discussed above by way of various examples, it should be understood that such details are provided merely for illustrative purposes. The appended claims are not limited to the disclosed embodiments; instead, the claims are intended to cover all modifications and equivalent combinations that fall within the spirit and scope of the embodiments of the present disclosure. For example, although the system components described above may be implemented through hardware devices, the same may also be achieved solely through software solutions, such as installing the described system on existing servers or mobile devices.

Similarly, it should be noted that in order to simplify the presentation of the disclosure of this specification, and thereby aid in the understanding of one or more embodiments of the invention, the foregoing descriptions of embodiments of this specification sometimes combine a variety of features into a single embodiment, accompanying drawings, or the description thereof. description thereof. However, this method of disclosure does not imply that the objects of the present specification require more features than those mentioned in the claims. Rather, claimed subject matter may lie in less than all features of a single foregoing disclosed embodiment.

Numbers describing the number of compositions, attributes are used in some embodiments, and it should be understood that such numbers used in the description of embodiments, in some examples, use the modifiers "about ", "approximately", or "generally" is used in some examples. Unless otherwise noted, the terms "about," "approximate," or "approximately" indicates that a $\pm 20\%$ variation in the stated number is allowed. Correspondingly, in some embodiments, the numerical parameters used in the specification and claims are approximations, which approximations are subject to change depending on the desired characteristics of the individual embodiment. In some embodiments, the numerical parameters should take into account the specified number of valid digits and use a general digit retention method. While the numerical domains and parameters used to confirm the breadth of their ranges in some embodiments of this specification are approximations, in specific embodiments such values are set to be as precise as possible within a feasible range.

For each of the patents, patent applications, patent application disclosures, and other materials cited in this specification, such as articles, books, specification sheets, publications, documents, and the like, the entire contents thereof are hereby incorporated herein by reference. Application history documents that are inconsistent with or conflict with the contents of this specification are excluded, as are documents (currently or hereafter appended to this specification) that limit the broadest scope of the claims of this specification. It should be noted that in the event of any inconsistency or conflict between the descriptions, definitions, and/or use of terms in the materials appended to this specification and those set forth herein, the descriptions, definitions and/or use of terms in this specification shall control. use shall prevail.

Finally, it should be understood that the embodiments described in this specification are used only to illustrate the principles of the embodiments of this specification. Other deformations may also fall within the scope of this specification. As such, alternative configurations of embodiments of the present specification may be viewed as consistent with the teachings of the present specification as an example, not as a limitation. Correspondingly, the embodiments of the present specification are not limited to the embodiments expressly presented and described herein.

**Claims**

1. A force feedback system, comprising:

    a glove body;
    a microprocessor coupled to the glove body, the microprocessor being communicatively coupled to an external computing device; and
    a plurality of finger force feedback components, each of the plurality of finger force feedback components being mechanically coupled to the glove body and communicatively coupled to the microprocessor, and being configured to provide, based on instructions from the microprocessor, force feedback to a finger corresponding to the finger force feedback component, wherein

    the finger force feedback component comprises a drawstring tracking a movement of the finger, a transmission member following a movement of the drawstring, and a stopping member,
    a plurality of stopping structures are sequentially disposed on the transmission member, and
    the stopping member is configured to cooperate with any one of the plurality of stopping structures to brake the finger.

2. The force feedback system of claim 1, wherein

    the finger force feedback component includes a housing located on a backside of a hand, the transmission member and the stopping member are disposed within the housing, and
    the force feedback system further includes a fingertip sleeve corresponding to the finger force feedback component, the fingertip sleeve being mechanically connected to the transmission member through the drawstring.

3. The force feedback system of claim 2, wherein

    the finger force feedback component also includes a wire groove disposed between finger joints of the glove body, and

the drawstring passes through the wire groove to connect the fingertip sleeve and the transmission member.

4. The force feedback system of claim 2, wherein

the transmission member comprises a spool, the drawstring is wrapped around the spool, a surface of the spool is provided with the plurality of stopping structures, and

the stopping member includes a stopping sheet that is capable of being inserted into a groove between the plurality of stopping structures.

5. The force feedback system of claim 4, wherein a gradient on one side of a stopping structure in the plurality of stopping structures that abuts the stopping sheet is greater than a gradient on the other side of the stopping structure.

6. The force feedback system of claim 4, wherein a tooth spacing of the plurality of stopping structures is less than 2 mm.

7. The force feedback system of claim 6, wherein the tooth spacing of the plurality of stopping structures is less than 1 mm.

8. The force feedback system of claim 4, wherein an inner surface of the housing that is opposite to the plurality of stopping structures is provided with a limiting protrusion.

9. The force feedback system of claim 8, wherein a height of the limiting protrusion is greater than a height of the plurality of stopping structures.

10. The force feedback system of claim 4, wherein the stopping member includes a transmission rod configured to press or release the stopping sheet in response to an instruction, wherein

when the transmission rod presses the stopping sheet, the stopping sheet is inserted into the groove between the plurality of stopping structures.

11. The force feedback system of claim 4, wherein the stopping member comprises a piezoelectric structure configured to drive the stopping sheet to insert into the groove between the plurality of stopping structures.

12. The force feedback system of claim 10 or 11, wherein the stopping sheet corresponds to a plurality of depths when inserted into the groove between the plurality of stopping structures.

13. The force feedback system of claim 1, wherein the finger force feedback component further includes a sensing member configured to detect a movement distance of the drawstring and transmit data relating to the movement distance of the drawstring to the microprocessor.

14. The force feedback system of claim 13, wherein

the transmission member comprises a spool, the drawstring is wrapped around the spool, and

the sensing member comprises a magnet mechanically connected to the spool and a magnetic field sensor configured to measure rotation information of the magnet as the spool rotates.

15. The force feedback system of claim 14, wherein the magnet is arranged coaxially with the magnetic field sensor along a central axis of a rotation of the spool.

16. The force feedback system of claim 14, wherein the magnet is mechanically connected to a side surface of the spool, and the side surface is arranged with a limiting protrusion.

17. The force feedback system of claim 16, wherein a height of the limiting protrusion is greater than a height of the magnet.

18. The force feedback system of claim 1, wherein the finger force feedback component further includes strain sensors, and the strain sensors are arranged at finger joints of the glove body and configured to read posture data of the finger.

19. The force feedback system of claim 1, wherein

the transmission member includes a spool and a spiral spring mechanically connected to the spool, the drawstring is wrapped around the spool, and

the spiral spring is configured to drive the spool to rotate in an opposite direction to reset the drawstring after the finger releases an external pulling force.

20. The force feedback system of claim 19, wherein a stiffness coefficient of the spiral spring is less than 100 N/m.

**100**

Glove body
110

Microprocessor
120

Finger force feedback
component 130

**FIG. 1**

Microprocessor
120

Wireless transmission module 210

Power supply module 230

Inertial sensing module 240

Data processing module 220

**FIG. 2**

External computing device 140

Sensing signal →

Microprocessor 120

← Control signal

Sensing signal →

Control instruction

Finger force feedback component 130

Sensing component 131

Stopping member 132

**FIG. 3**

**400**

**FIG. 4A**

**400**

**FIG. 4B**

**FIG. 5A**

**FIG. 5B**

**FIG. 6A**

**FIG. 6B**

**FIG. 7A**

**FIG. 7B**

**FIG. 8**

**FIG. 9**

**FIG. 10**

**1100**

Z-axis
Y-axis
X-axis
1120
1110
1130

**FIG. 11**

**1100**

Z-axis
Y-axis
X-axis
1120
1110
1130

**FIG. 12**

**1100**

**FIG. 13A**

**FIG. 13B**

**FIG. 14A**

**FIG. 14B**

**FIG. 15**

**FIG. 16**

**FIG. 17**

## INTERNATIONAL SEARCH REPORT

| International application No. |
| --- |
| **PCT/CN2024/099232** |

### A. CLASSIFICATION OF SUBJECT MATTER

G01B 7/16(2006.01)i

According to International Patent Classification (IPC) or to both national classification and IPC

### B. FIELDS SEARCHED

Minimum documentation searched (classification system followed by classification symbols)

IPC: G01B, G06F, A61H, A61F, A61B

Documentation searched other than minimum documentation to the extent that such documents are included in the fields searched

Electronic data base consulted during the international search (name of data base and, where practicable, search terms used)

CNTXT, ENTXTC, DWPI, WPABS: 缠绕, 齿, 传感器, 磁, 带, 定位, 霍尔, 棘轮, 距离, 卷簧, 拉绳, 力反馈, 扭簧, 扭转弹簧, 绳, 手套, 丝, 锁定, 锁止, 位移, 涡卷弹簧, 限位, 线, 线轴, 止动, 深度, 深浅, 多, 不同, 位置, feedback, glove?, rope?, fix+, ratchet, gear, magnetic+

### C. DOCUMENTS CONSIDERED TO BE RELEVANT

| Category* | Citation of document, with indication, where appropriate, of the relevant passages | Relevant to claim No. |
| --- | --- | --- |
| X | CN 114779943 A (XUE YUAN et al.) 22 July 2022 (2022-07-22) description, paragraphs 81-164, and figures 1-11 | 1-3, 13, 18 |
| Y | CN 114779943 A (XUE YUAN et al.) 22 July 2022 (2022-07-22) description, paragraphs 81-164, and figures 1-11 | 4-11, 19-20 |
| Y | CN 113616210 A (HUAZHONG UNIVERSITY OF SCIENCE AND TECHNOLOGY) 09 November 2021 (2021-11-09) description, paragraphs 34-43, and figures 1-10 | 4-11, 19-20 |
| A | CN 103659825 A (TSINGHUA UNIVERSITY) 26 March 2014 (2014-03-26) entire document | 1-20 |
| A | CN 108371610 A (HUAZHONG UNIVERSITY OF SCIENCE AND TECHNOLOGY et al.) 07 August 2018 (2018-08-07) entire document | 1-20 |
| A | CN 115390662 A (EAST CHINA NORMAL UNIVERSITY) 25 November 2022 (2022-11-25) entire document | 1-20 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **23 August 2024** | **29 August 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/099232**

### C.    DOCUMENTS CONSIDERED TO BE RELEVANT

| Category* | Citation of document, with indication, where appropriate, of the relevant passages | Relevant to claim No. |
|---|---|---|
| A | CN 114029990 A (HUA HONGWEI) 11 February 2022 (2022-02-11)<br>entire document | 1-20 |
| A | CN 207301976 U (GOERTEK TECHNOLOGY CO., LTD.) 01 May 2018 (2018-05-01)<br>entire document | 1-20 |
| A | CN 108371575 A (PUTIAN UNIVERSITY) 07 August 2018 (2018-08-07)<br>entire document | 1-20 |
| A | JP 2001165268 A (SONY CORP.) 19 June 2001 (2001-06-19)<br>entire document | 1-20 |

Form PCT/ISA/210 (second sheet) (July 2022)

**INTERNATIONAL SEARCH REPORT**
Information on patent family members

International application No.

**PCT/CN2024/099232**

| Patent document cited in search report | | | Publication date (day/month/year) | Patent family member(s) | | | Publication date (day/month/year) |
|---|---|---|---|---|---|---|---|
| CN | 114779943 | A | 22 July 2022 | None | | | |
| CN | 113616210 | A | 09 November 2021 | None | | | |
| CN | 103659825 | A | 26 March 2014 | None | | | |
| CN | 108371610 | A | 07 August 2018 | None | | | |
| CN | 115390662 | A | 25 November 2022 | None | | | |
| CN | 114029990 | A | 11 February 2022 | None | | | |
| CN | 207301976 | U | 01 May 2018 | None | | | |
| CN | 108371575 | A | 07 August 2018 | None | | | |
| JP | 2001165268 | A | 19 June 2001 | JP | 4389135 | B2 | 24 December 2009 |

Form PCT/ISA/210 (patent family annex) (July 2022)

**EP 4 653 805 A1**

## REFERENCES CITED IN THE DESCRIPTION

### Patent documents cited in the description

*   CN 2023124295 W